# EUROPEAN PATENT APPLICATION

(11) **EP 4 534 103 A1**
(43) Date of publication of application: **09.04.2025**
(21) Application number: 23815125.2
(22) Date of filing: 29.05.2023
(51) Int. Cl.: A61K 47/68, A61K 31/4745, A61K 31/537, A61K 38/07, A61K 45/00, A61K 45/06, A61P 35/00, C07K 16/28

(54) **PHARMACEUTICAL COMPOSITION OF RECOMBINANT ANTI-HUMAN CLDN18.2 MONOCLONAL ANTIBODY-MMAE CONJUGATE**

(30) Priority: 31.05.2022 CN 202210606878
(71) Applicant: CSPC Megalith Biopharmaceutical Co., Ltd., Shijiazhuang, Hebei 050025 (CN)
(72) Inventor: XIE, Ru, Shijiazhuang, Hebei 050025 (CN); HAN, Weiqiang, Shijiazhuang, Hebei 050025 (CN); SU, Li, Shijiazhuang, Hebei 050025 (CN); CAO, Weirong, Shijiazhuang, Hebei 050025 (CN); HUI, Xiwu, Shijiazhuang, Hebei 050025 (CN); LIU, Boning, Shijiazhuang, Hebei 050025 (CN); YAO, Bing, Shijiazhuang, Hebei 050025 (CN); WANG, Chao, Shijiazhuang, Hebei 050025 (CN)
(74) Representative: Patentanwälte Olbricht Buchhold Keulertz
(86) International application number: PCT/CN2023/096728
(87) International publication number: WO 2023/231942

(57) **Abstract**

The present application provides a pharmaceutical composition, comprising an anti-CLDN18.2 antibody-drug conjugate, a buffer, a stabilizer and a surfactant. The present application further provides a method for preparing the pharmaceutical composition and use of the pharmaceutical composition.

## Description

### Cross-Reference to Related Applications

The present application claims the right of priority for Chinese patent application no. 202210606878.4, titled "PHARMACEUTICAL COMPOSITION OF RECOMBINANT ANTI-HUMAN CLDN18.2 MONOCLONAL ANTIBODY-MMAE CONJUGATE" and filed on May 31, 2022, the entire content of which is incorporated herein by reference in its entirety for all purposes.

### Technical Field

The present application generally relates to the field of biomedicine, and specifically relates to a pharmaceutical composition of an antibody-drug conjugate, a method for preparing the pharmaceutical composition, and the use of the pharmaceutical composition.

### Background Art

Claudins are a family of proteins that are widely present in epithelial and endothelial cells of vertebrates, and function to build intercellular barriers to control molecular flow between cells. CLDN18.2 (Claudin 18.2), one of the most studied members of the Claudin protein family, is normally expressed only in gastric epithelial cells, with a high degree of tissue specificity. When carcinogenic, Claudin18.2 is highly expressed in various tumor tissues, such as gastric cancer (60% to 80%), pancreatic cancer (50%), esophageal cancer (30% to 50%) and lung cancer (40% to 60%). Claudin18.2 protein is normally buried in the cell membrane of epithelial cells, and malignant tumors destroy tight junctions, exposing Claudin18.2 on the surface of tumor cells to be an attackable target, so that Claudin18.2 becomes a new target with great potential for targeted therapy and immunotherapy.

Gastric cancer ranks third in cancer-related mortality, and is considered as one of the most refractory cancers worldwide. In patients with advanced or metastatic gastric cancer or gastroesophageal junction (GEJ) adenocarcinoma, the median overall survival (mOS) does not exceed 10 months. Compared with the target human epidermal growth factor receptor 2 (HER2), the number of patients having the target Claudin18.2 accounts for approximately 50% to 60% of all patients with gastric cancer, making the treatment of the target Claudin18.2 extremely potential. Current product types that target the target Claudin 18.2 worldwide encompass various types such as monoclonal antibodies, bispecific antibodies, CAR-T and ADC, including the predominant product forms at present.

There is an urgent need in the art for anti-cancer product that targets Claudin 18.2.

### Summary of the Invention

In a first aspect, the present application provides a pharmaceutical composition comprising an anti-CLDN18.2 antibody-drug conjugate, a buffer, a stabilizer and a surfactant, wherein the antibody comprises a heavy chain comprising HCDR1 to HCDR3 having amino acid sequences as shown in SEQ ID NOs: 1, 2 and 3, respectively, and/or a light chain comprising LCDR1 to LCDR3 having amino acid sequences as shown in SEQ ID NOs: 4, 5 and 6, respectively, and the structure of the drug-linker moiety (L&D) of the antibody-drug conjugate is as follows:

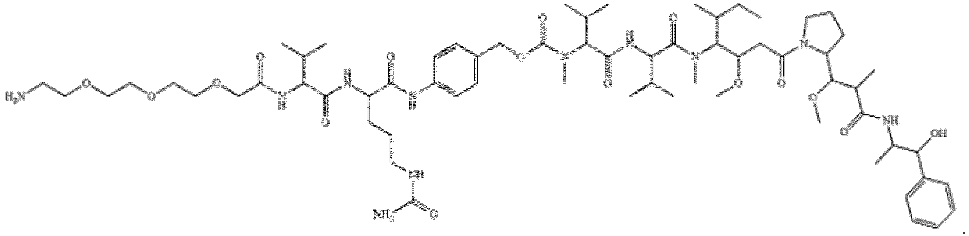

In a second aspect, the present application provides the use of the pharmaceutical composition of the first aspect in the preparation of a drug for the treatment of a cancer.

In a third aspect, the present application provides a method for treating a cancer, wherein the method comprises administering to an individual having a cancer in need thereof an effective amount of the pharmaceutical composition of the first aspect.

In a fourth aspect, the present application provides a method for preparing the pharmaceutical composition of the first aspect, wherein the method comprises the following steps:
preparing the anti-CLDN18.2 antibody-drug conjugate,
transferring the anti-CLDN18.2 antibody-drug conjugate to a liquid system containing the other components of the pharmaceutical composition by liquid replacement, and
performing sterile filtration and sterile filling.

### Brief Description of the Drawings

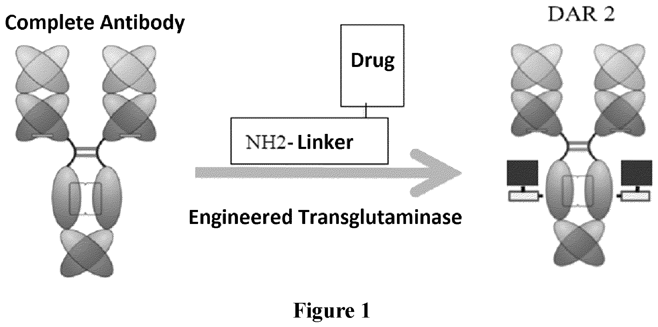

FIG. 1 shows a schematic diagram of the conjugation reaction for ADC synthesis using mTgase method.

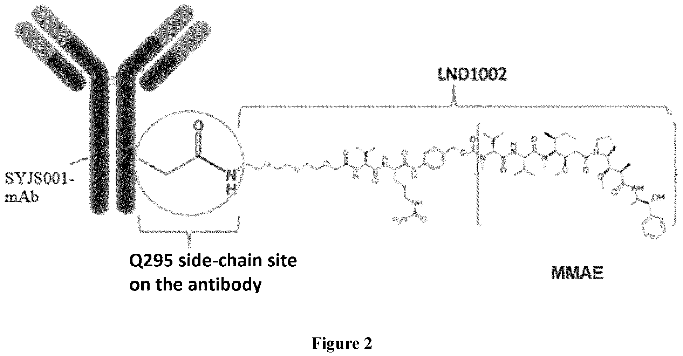

FIG. 2 shows a schematic diagram of the structure of SYJS001-ADC, where LND1002 represents a linker (Linker) + drug molecule (Drug) moiety, LND1002 shows the complete structure, and the circle-marked part represents the amide bond linkage between the linker in LND1002 and the antibody. The SYJS001-ADC disclosed in the present application is a site-specific antibody-drug conjugate. Each molecule is formed by conjugating one molecule of MMAE to the amino acid at position Q298 (i.e., at Q295 according to Kabat numbering) of each heavy chain of one anti-CLDN18.2 monoclonal antibody via a linker (NH₂-PEG₃-Val-Cit). The antibody is linked to the linker via a stable amide bond (isopeptide bond) with the mean drug-to-antibody ratio (DAR) of 2.0.
FIG. 3 shows curves of binding of SYJS001-ADC to cells expressing human, murine and monkey CLDN18.2.
FIG. 4 shows the cross-reactivity experiment results of SYJS001 monoclonal antibody (the monoclonal antibody used in SYJS001-ADC).
FIG. 5 shows the experimental results of specific binding of SYJS001 monoclonal antibody and SYJS001-ADC to CLDN18.2 protein.
FIG. 6 shows the in vivo tumor inhibition effect of SYJS001-ADC in BxPC-3-CLDN18.2 tumor-bearing mouse models (compared to gemcitabine).
FIG. 7 shows the in vivo tumor inhibition effect of SYJS001-ADC in NUGC4-CLDN18.2 tumor-bearing mouse models (compared to cisplatin).
FIG. 8 shows the in vivo tumor inhibition effect of SYJS001-ADC in BxPC-3-CLDN18.2 tumor-bearing mouse models (compared to IMAB362-ADC).
FIG. 9 shows the in vivo tumor inhibition effect of SYJS001-ADC in NUGC4-CLDN18.2 tumor-bearing mouse models (compared to IMAB362-ADC).

### Detailed Description of the Invention

### Definitions

Unless otherwise defined, all scientific and technical terms used herein have the same meaning as understood by those of ordinary skill in the art. For definitions and terms in the art, professionals can refer to Current Protocols in Molecular Biology (Ausubel). Abbreviations for amino acid residues are the standard 3-letter and/or 1-letter codes used in the art to refer to one of the 20 commonly used L-amino acids.

Although the numerical ranges and parameters as shown in the broad scope of the present application are approximations, the numerical values as shown in specific embodiments are recited as precisely as possible. However, any numerical value inherently contains certain errors resulting from the standard deviation found in their respective measurements. Moreover, all ranges disclosed herein shall be understood to encompass any and all subranges subsumed therein. For example, the recited range "1 to 10" shall be considered to comprise any and all subranges between the minimum value of 1 and the maximum value of 10 (including endpoint values), i.e., all subranges that start with the minimum value of 1 or greater, such as 1 to 6.1, and subranges with the maximum value of 10 or less, such as 5.5 to 10. Additionally, any reference document referred to as "incorporated herein" shall be understood to be incorporated in its entirety.

The term "antibody-drug conjugate" used herein, also referred to as "ADC", refers to a cytotoxic drug conjugated to an antibody. In some ADCs that target cancer cells, the antigen targeted by the antibody can be expressed on the surface of the cancer cells, and the antibody also binds to the antigen that can be internalized in the cells and therefore can selectively deliver a drug to the cancer cells, thereby causing the drug to accumulate in the cancer cells and kill the cancer cells.

The term "pharmaceutical composition" used herein refers to a combination of at least one drug and optionally a pharmaceutically acceptable carrier or excipient that are combined together to achieve a specific purpose. The pharmaceutically acceptable carrier is water, an aqueous buffer solution, an isotonic saline solution such as PBS (phosphate buffer), glucose, mannitol, dextrose, lactose, starch, magnesium stearate, cellulose, magnesium carbonate, 0.3% glycerin, hyaluronic acid, ethanol or polyalkylene glycol such as polypropylene glycol and triglyceride. The type of the pharmaceutically acceptable carrier used particularly depends on whether the composition according to the present application is prepared for use in oral, nasal, intradermal, subcutaneous, intramuscular or intravenous administration. The composition according to the present application may comprise a wetting agent, an emulsifying agent or a buffer substance as an additive.

The pharmaceutical composition according to the present application can be administered by any suitable route, such as oral administration, nasal administration, intradermal administration, subcutaneous administration, intramuscular administration or intravenous administration.

The "therapeutically effective amount" or "effective amount" used herein refers to a dose sufficient to show the benefit to a subject to whom a drug is administered. The actual amount of administration and the rate and time course of administration, will depend on the individual condition and disease severity of a person to be treated. The prescription of treatment (e.g., decisions on the dose) is ultimately the responsibility of and depends on general practitioners and other physicians, generally considering the disease to be treated, the condition of an individual patient, the site of delivery, the method of administration and other factors known to the physicians.

The term "subject" or "individual" used herein refers to mammals, such as humans, but may also be other animals, such as wild animals (such as herons, storks and cranes), domestic animals (such as ducks and geese) or experimental animals (such as orangutans, monkeys, rats, mice, rabbits, guinea pigs, groundhogs and ground squirrels).

The term "antibody" broadly encompasses an intact antibody and any antigen-binding fragment ("antigen-binding moiety") or single chain form thereof. The "full-length/intact antibody" refers to a protein comprising at least two heavy (H) chains and two light (L) chains interlinked by disulfide bonds. Each heavy chain comprises a heavy chain variable region (abbreviated as VH) and a heavy chain constant region comprising three domains, CH1, CH2 and CH3. Each light chain comprises a light chain variable region (abbreviated as VL) and a light chain constant region comprising one domain, CL. The VH and VL regions can also be subdivided into multiple highly variable regions, called complementarity determining regions (CDRs), which are interspersed with multiple more conserved regions called framework regions (FRs). VH and VL each are composed of three CDRs and four FRs, which are arranged from an amino terminus to a carboxyl terminus in the following order: FR1, CDR1, FR2, CDR2, FR3, CDR3, FR4. These variable regions of the heavy and light chains comprise binding domains that interact with an antigen. The constant region of an antibody can mediate the binding of the immunoglobulin to the tissue or factor of a host, comprising various cells (e.g., effector cells) of the immune system and the first component (Clq) of the classical complement system. Chimeric or humanized antibodies are also encompassed in the antibodies according to the present application. The full-length/intact antibody may be antibodies of any class, such as IgD, IgE, IgG, IgA or IgM (or the subclasses of the above-mentioned antibodies), but the antibody does not need to be of any particular class. Immunoglobulins can be designated into different classes according to the amino acid sequence of the heavy chain constant domain in the antibody. Generally, there are five main classes of immunoglobulins: IgA, IgD, IgE, IgG and IgM, and several of these classes can be further divided into subclasses (isotypes), such as IgG1, IgG2, IgG3, IgG4, IgA1 and IgA2. The heavy chain constant domains that correspond to the different classes of immunoglobulins are referred to as α, δ, ε, γ and µ, respectively. The subunit structures and three-dimensional structures of different classes of immunoglobulins are well known.

The complementarity determining regions (CDRs, generally including CDR1, CDR2 and CDR3) are the regions in the variable regions that have the greatest effect on the affinity and specificity of an antibody. There are a variety of common definition methods for the CDR sequences of VH or VL, including IMGT, Chothia definition and Kabat definition. Embodiments of the present application can determine the CDR sequences in VH and VL sequences according to the Kabat definition.

The term "humanized antibody" refers to an antibody that may comprise CDR regions derived from an antibody of human origin, and the other parts of the antibody molecule are derived from one (or several) human antibodies. Moreover, in order to retain the binding affinity, some residues of the framework (referred to as FR) segment can be modified. The humanized antibodies or the fragments thereof according to the present application can be prepared by techniques known to those skilled in the art.

The term "chimeric antibody" refers to an antibody in which the variable region sequences are from one species and the constant region sequences are from another species, e.g., an antibody in which the variable region sequences are from a mouse antibody and the constant region sequences are from a human antibody. The chimeric antibody or the fragment thereof according to the present application can be prepared using genetic recombination techniques. For example, the chimeric antibody can be produced by cloning a recombinant DNA comprising a promoter, a sequence encoding the variable region of the non-human monoclonal antibody, especially the murine monoclonal antibody, according to the present application, and a sequence encoding the constant regions of a human antibody. The chimeric antibody of the present application encoded by such recombinant gene will be, for example, a mouse-human chimera. The specificity of the antibody is determined by the variable region derived from the murine DNA, and the isotype of the antibody is determined by the constant regions derived from the human DNA. For methods for preparing chimeric antibodies, for example, reference can be made to the document Verhoeyn et al. (BioEssays, 8:74, 1988).

The term "monoclonal antibody" refers to a preparation of a single-component antibody molecule. The monoclonal antibody composition exhibits single binding specificity and affinity to a particular epitope.

According to the structural information of the anti-CLDN18.2 monoclonal antibody given in the present application, the antibody can be prepared in CHO-K1 cells (ATCC Number: CCL-61, Lot No.: 59965043) using methods known in the art.

The term "specifically bind" refers to a non-random binding reaction between two molecules, such as the binding of an antibody to an antigenic epitope.

The terms "transglutaminase" and "TGase" are used interchangeably herein and refer to an enzyme that can be used to perform a transglutamination reaction. The term "transglutamination" used herein refers to a reaction where the γ-glutaminyl of an acceptor glutamine residue from a protein/peptide is transferred to an amine group, such as a primary amine or the ε-amino group of lysine.

The inventors of the present application study and explore the pharmaceutics of antibody-drug conjugates/ADCs to develop various inventions of the present application.

In the studies of antibody preparations, the formation of aggregates and the generation of decomposition products cause pharmaceutically undesirable side effects, which result in an increased immunogenicity or risk associated with venous disorders in patients receiving the drug treatment. On the basis of the above-mentioned reasons, when antibodies are prepared, it is necessary to inhibit the formation of aggregates and the generation of decomposition products, and in view of this, researchers have studied various preparation forms (e.g., aqueous injection forms and freeze-dried injection forms) of pharmaceutical compositions and the formulas of the preparations. Similarly, there will be more technical problems when the pharmaceutical preparations of antibody-drug conjugates are studied. Not only the specific properties of the antibody moiety need to be considered, but also the specific properties of the drug-linker moiety need to be considered. For example, the antibody-drug conjugate may shed small-molecule toxins during storage, which may affect the efficacy and toxicity of the related drug.

One of the technical objectives of the present application is to provide a pharmaceutical composition (especially in the form of an aqueous injection) of an antibody-drug conjugate that can inhibit the formation of aggregates and the generation of decomposition products and reduce the shedding of toxins, and a related preparation method.

As an example, the inventors of the present application perform specific site-directed modification (Q295) on glutamine on the heavy chain of the self-developed recombinant anti-human CLDN18.2 monoclonal antibody under the catalysis of microbial transglutaminase (mTgase) and conjugating of the modified antibody to a small-molecule tubulin inhibitor drug MMAE to obtain a related antibody-drug conjugate, with the mean drug-to-antibody ratio (DAR) of 2.0, in which the DAR2 distribution is greater than 70%, indicating high product uniformity. On this basis, the liquid preparation formulas that are particularly suitable for the above-mentioned antibody-drug conjugate are prepared. The technical advantages achieved include at least one of the followings: on the premise of meeting the requirement of stability, the production cost can be significantly reduced, the production process can be shortened, the administration is more convenient, and disadvantages such as complex redissolution procedures during the use of freeze-dried drugs, and high requirements for the technical level of preparation personnel can be avoided.

In a first aspect, the present application provides a pharmaceutical composition comprising an antibody-drug conjugate, a buffer, a stabilizer and a surfactant.

In some embodiments, the structure of the drug & linker moiety (L&D) in the antibody-drug conjugate is as shown in formula I below:

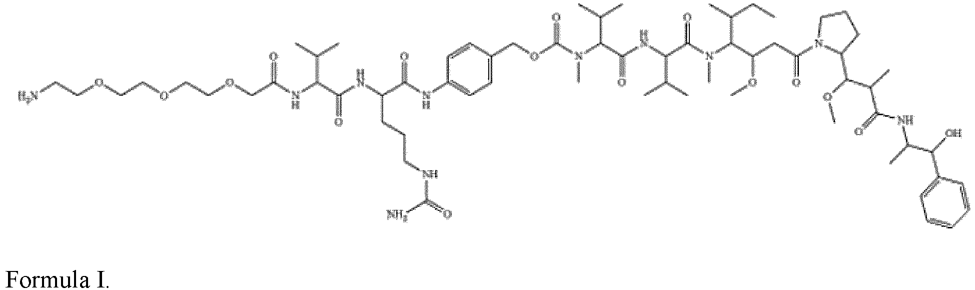

In some embodiments, the antibody in the antibody-drug conjugate is an anti-CLDN18.2 antibody.

In some embodiments, the antibody comprises a heavy chain comprising HCDR1 to HCDR3 having amino acid sequences as shown in SEQ ID NOs: 1, 2 and 3, respectively, and/or a light chain comprising LCDR1 to LCDR3 having amino acid sequences as shown in SEQ ID NOs: 4, 5 and 6, respectively. In some embodiments, the heavy chain comprises a heavy chain variable region having an amino acid sequence as shown in SEQ ID NO: 7, and/or the light chain comprises a light chain variable region having an amino acid sequence as shown in SEQ ID NO: 8. In some embodiments, the antibody comprises a heavy chain and a light chain having amino acid sequences as shown in SEQ ID NO: 9 and SEQ ID NO: 10, respectively. In some embodiments, the antibody is a monoclonal antibody or a bispecific antibody. In some embodiments, the antibody is a monoclonal antibody. In some embodiments, the antibody is a humanized antibody. In some embodiments, the antibody is a fully human antibody. In some specific embodiments, the antibody has ADCC activity. In some specific embodiments, the antibody has CDC activity. In some specific embodiments, the antibody specifically binds to CLDN18.2 and does not substantially bind to CLDN18.1. In some specific embodiments, the antibody is an IgG1κ antibody.

In some embodiments, the buffer includes, but not limited to, acetate, succinate, gluconate, histidine salt, oxalate, lactate, phosphate, citrate, tartrate, fumarate, glycylglycine and other organic acid buffers.

In some embodiments, the histidine salt buffer is a buffer containing histidine ions. Examples of histidine salt buffers include histidine-histidine hydrochloride, histidine-acetate, histidine-phosphate, histidine-sulfate and other buffers, wherein the histidine-acetate buffer is prepared from histidine and acetic acid, and the histidine-histidine hydrochloride buffer is prepared from histidine and histidine hydrochloride. Similarly, the succinate buffer may be succinic acid-sodium succinate, and the citrate buffer may be citric acid-sodium citrate.

In some embodiments, the buffer is a histidine-histidine hydrochloride buffer.

In some embodiments, the stabilizer includes, but not limited to, a sugar (such as sucrose and trehalose), a polyol (such as mannitol and sorbitol), and an amino acid (L-serine, sodium glutamate, alanine, glycine, sarcosine, etc.).

In some embodiments, the surfactant includes, but not limited to, polysorbate 20 or polysorbate 80.

In some embodiments, the pharmaceutical composition has a pH of 5.0-7.4. In some embodiments, the pharmaceutical composition has a pH of 5.0-6.5. In some embodiments, the pharmaceutical composition has a pH of 5.5.

In some embodiments, the pharmaceutical composition further comprises water.

In some embodiments, the components of the pharmaceutical composition independently or collectively comprise:
(a) histidine-histidine hydrochloride, with the content of 5-30 mM, such as about 5 mM, about 6 mM, about 7 mM, about 8 mM, about 9 mM, about 10 mM, about 11 mM, about 12 mM, about 13 mM, about 14 mM, about 15 mM, about 16 mM, about 17 mM, about 18 mM, about 19 mM, about 20 mM, about 21 mM, about 22 mM, about 23 mM, about 24 mM, about 25 mM, about 26 mM, about 27 mM, about 28 mM, about 29 mM and about 30 mM,
(b) polysorbate 20 or polysorbate 80, with the content of 0.02 wt% to 0.08 wt%, such as about 0.02 wt%, about 0.03 wt%, about 0.04 wt%, about 0.05 wt%, about 0.06 wt%, about 0.07 wt% and about 0.08 wt%,
(c) sucrose, with the content of 6 wt% to 8 wt%, such as about 6 wt%, 6.5 wt%, 7 wt%, 7.5 wt% and 8 wt%, and
(d) an antibody-drug conjugate, with the content of 5-50 mg/ml, such as about 5 mg/ml, about 10 mg/ml, about 15 mg/ml, about 20 mg/ml, about 25 mg/ml, about 30 mg/ml, about 35 mg/ml, about 40 mg/ml, about 45 mg/ml and about 50 mg/ml, and has
(e) a pH of 5.0-6.5, such as about 5.0, about 5.5, about 6.0 and about 6.5.

In some embodiments, the pharmaceutical composition comprises:
(a) 5-30 mM histidine-histidine hydrochloride,
(b) 0.02 wt% to 0.08 wt% of polysorbate 20 or polysorbate 80,
(c) 6 wt% to 8 wt% of sucrose and
(d) 5-50 mg/ml antibody-drug conjugate, and has
(e) a pH of 5.0-6.5.

In some embodiments, the pharmaceutical composition comprises:
(a) 10-30 mM histidine-histidine hydrochloride,
(b) 0.02 wt% to 0.04 wt% of polysorbate 20 or polysorbate 80,
(c) 6 wt% to 7 wt% of sucrose and
(d) 10-20 mg/ml antibody-drug conjugate, and has
(e) a pH of 5.0-6.0.

In some embodiments, the pharmaceutical composition comprises:
(a) 20 mM histidine-histidine hydrochloride,
(b) 0.02 wt% of polysorbate 20,
(c) 6 wt% of sucrose and
(d) 10 mg/ml antibody-drug conjugate, and has
(e) a pH of 5.5.

In some embodiments, the pharmaceutical composition can be prepared as a freeze-dried preparation, a liquid preparation or a powder injection preparation.

In some embodiments, the pharmaceutical composition of the present application is used for the treatment of a cancer. In some embodiments, the cancer is a CLDN18.2-positive cancer.

In some embodiments, the cancer is the one in which CLDN18.2 is expressed on the surface of the cancer cells. In some embodiments, the cancer is a cancer that highly expresses CLDN18.2 (CLDN18.2+). In some embodiments, the cancer that highly expresses CLDN18.2 (CLDN18.2+) means that at least 60% of the cancer cells in the cancer cell population express CLDN18.2. In some embodiments, the cancer that highly expresses CLDN18.2 (CLDN18.2+) means that at least 70% of the cancer cells in the cancer cell population express CLDN18.2. In some embodiments, the cancer that highly expresses CLDN18.2 (CLDN18.2+) means that at least 80% of the cancer cells in the cancer cell population express CLDN18.2. In some embodiments, the cancer that highly expresses CLDN18.2 (CLDN18.2+) means that at least 90% of the cancer cells in the cancer cell population express CLDN18.2. In some embodiments, the cancer that highly expresses CLDN18.2 (CLDN18.2+) means that at least 95% of the cancer cells in the cancer cell population express CLDN18.2. In some embodiments, the cancer that highly expresses CLDN18.2 (CLDN18.2+) means that at least 98% of the cancer cells in the cancer cell population express CLDN18.2. In some embodiments, the cancer that highly expresses CLDN18.2 (CLDN18.2+) means that at least 99% of the cancer cells in the cancer cell population express CLDN18.2.

In some embodiments, the pharmaceutical composition of the present application can be used for the treatment of at least one cancer selected from the following cancers: breast cancer, gastric cancer (also known as gastric adenocarcinoma), colorectal cancer (also known as colon and rectal cancer, and including colon cancer and rectal cancer), lung cancer (including small cell lung cancer and non-small cell lung cancer), esophageal cancer, salivary gland cancer, esophagogastric junction adenocarcinoma, cholangiocarcinoma, Paget's disease, pancreatic cancer, ovarian cancer, uterine carcinosarcoma, urothelial carcinoma, prostate cancer, bladder cancer, gastrointestinal stromal tumor, gastrointestinal stromal tumor, cervical cancer, squamous cell carcinoma, peritoneal cancer, liver cancer, hepatocellular carcinoma, colon cancer, rectal cancer, endometrial cancer, uterine cancer, kidney cancer, vulvar cancer, thyroid cancer, penile cancer, leukemia, malignant lymphoma, plasmacytoma, myeloma, neuroepithelial tissue tumor, schwannoma, head and neck cancer, skin cancer, laryngeal cancer, gallbladder cancer, cholangiocarcinoma, mesothelioma and sarcoma.

In some embodiments, the pharmaceutical composition of the present application can be used for the treatment of at least one cancer selected from the following cancers: breast cancer, gastric cancer, colorectal cancer, non-small cell lung cancer, esophageal cancer, salivary gland cancer, esophagogastric junction adenocarcinoma, cholangiocarcinoma, Paget's disease, pancreatic cancer, ovarian cancer and uterine carcinosarcoma.

In some embodiments, the pharmaceutical composition of the present application can be used for the treatment of at least one cancer selected from the following cancers: breast cancer, gastric cancer, colorectal cancer, non-small cell lung cancer, esophageal cancer, salivary gland cancer, esophagogastric junction adenocarcinoma, cholangiocarcinoma and Paget's disease.

In some embodiments, the pharmaceutical composition of the present application can be used for the treatment of breast cancer, gastric cancer, colorectal cancer or non-small cell lung cancer.

In some embodiments, the pharmaceutical composition of the present application can be selectively used as an agent for drug therapy, which is a main method for treating a cancer and therefore can delay the development of the cancer cells, inhibit the growth of the cancer cells and further kill the cancer cells. These effects can make patients with cancer free of symptoms caused by cancer, or achieve an improvement in the QOL (quality of life score) of patients with cancer, and obtain the therapeutic effects by maintaining the lives of patients with cancer. Even if the pharmaceutical composition and treatment method of the present application do not kill cancer cells, they can achieve a higher QOL (quality of life score) for patients with cancer by inhibiting or controlling the growth of cancer cells while achieving longer survival.

In some embodiments, the pharmaceutical composition of the present application can be used alone or in combination with other therapies such as a surgery, a radiation therapy and a hormonal therapy.

In some embodiments, the pharmaceutical composition of the present application can be administered in combination with other anti-cancer agents, thereby enhancing the anti-cancer effect. Examples of other anti-cancer agents for such purposes include 5-fluorouracil (5-FU), pertuzumab, trastuzumab, paclitaxel, carboplatin, cisplatin, gemcitabine, capecitabine, irinotecan (CPT-11), docetaxel, pemetrexed, sorafenib, vinblastine, vinorelbine, everolimus, tanespimycin, bevacizumab, oxaliplatin and lapatinib, but are not limited thereto, as long as they are agents having anti-tumor activity.

In a second aspect, the present application provides the use of the pharmaceutical composition of the first aspect in the preparation of a drug for the treatment of a cancer.

In a third aspect, the present application provides a method for treating a cancer, wherein the method comprises administering to an individual having a cancer in need thereof an effective amount of the pharmaceutical composition of the first aspect.

The embodiments and technical features in the first aspect with regard to the medical use of the pharmaceutical composition of the present application are also applicable to the second and third aspects.

In a fourth aspect, the present application provides a method for preparing the pharmaceutical composition of the first aspect, wherein the method comprises the following steps:
preparing the anti-CLDN18.2 antibody-drug conjugate,
transferring the anti-CLDN18.2 antibody-drug conjugate to a liquid system containing the other components of the pharmaceutical composition by liquid replacement, and
performing sterile filtration and sterile filling.

In some specific embodiments, the method for preparing the pharmaceutical composition of the present application comprises the following steps:
(1) preparing an aqueous solution comprising predetermined amounts of (i) an antibody-drug conjugate, (ii) a histidine buffer, (iii) sucrose and (iv) a surfactant; (2) if necessary, adjusting the pH of the aqueous solution to a predetermined value; and (3) performing sterile filtration, and subpackaging the resulting product into injection bottles.

In some embodiments, the method for preparing a pharmaceutical composition of the present application comprises the following steps:
(1) replacement of antibody-drug conjugate by ultrafiltration: preparing a histidine-histidine hydrochloride solution with the same concentration as the formula amount of the final product, performing liquid replacement using a 30 kD ultrafiltration membrane pack to replace the original affinity chromatography solution with the histidine-histidine hydrochloride solution, wherein the liquid replacement fold using ultrafiltration is no less than 100, the pH of the replacement liquid is controlled to be consistent with that of the final product, while the concentration of the antibody-drug conjugate is not lower than the concentration of the finished product (optimally, ≥ 1.2 times);
(2) preparation of semi-finished product diluent: according to the formula amount of the product and the concentration of the antibody-drug conjugate liquid obtained after liquid replacement using ultrafiltration, calculating the preparation amount of the final product, while calculating the required amount of various auxiliary materials and preparing a semi-finished product diluent; adding about 80% of water for injection into a preparation container, weighing histidine, histidine hydrochloride, polysorbate 20 and sucrose in sequence, adding the components into the preparation container, and stirring and mixing the components; and after the auxiliary materials in each group are completely dissolved, adding water for injection to adjust the volume to the preparation amount, stirring and mixing the resulting solution until uniform, and then filtering the resulting mixture for later use; and
(3) preparation of finished product: adding the prepared semi-finished product diluent to the prepared solution containing the antibody-drug conjugate liquid obtained after liquid replacement using ultrafiltration, using the diluent to adjust the volume to the preparation amount of the semi-finished product, and stirring and mixing the resulting solution until uniform; and performing sampling, detecting the protein content, the pH value and the microbial limits, performing sterile filtration, then subpackaging the products into sterilized injection bottles according to the specifications of the products, and plugging and capping the bottles.

In a fifth aspect, the present application further provides a freeze-dried preparation prepared by freeze-drying the liquid pharmaceutical composition prepared in the fourth aspect. In some embodiments, the method for preparing the freeze-dried preparation comprises the steps of pre-freezing, vacuuming, primary drying and vacuum drying.

### Examples:

The present application is further described below in conjunction with specific examples. It should be understood that these examples are merely used for describing the present application, rather than limiting the scope of the present application. In the following examples, conventional conditions or conditions suggested by the manufacturers are generally used, unless specific conditions are indicated in an experimental method.

### Example 1: Preparation and efficacy testing of anti-CLDN18.2 antibody-drug conjugate

### 1.1 Preparation of anti-CLDN18.2 antibody-drug conjugate

A certain volume of LND1002 (the structure was as shown in FIG. 2), a reaction buffer, a CLDN18.2 monoclonal antibody (see Table 30 for the exemplary antibody structure sequences used in this example, a vector containing heavy and light chain-related sequences could be expressed in a host cell using conventional technical means in the art, such as genetic engineering techniques, and the monoclonal antibody can be obtained by purification), mTGase (transglutaminase) and H₂O were transferred to a flexible ethylene-vinyl acetate single-use reaction bag by a peristaltic pump in an appropriate order. The reaction bag was sealed, and the components were mixed until uniform and then reacted at 30°C for 24-144 h. In the reaction process, samples were taken every 24 h and analyzed and detected for conjugation rate by C4-HPLC. When the conjugation rate >= 95%, the reaction was terminated and the reaction product was purified immediately to obtain the antibody-drug conjugate SYJS001. The overall structure of the antibody-drug conjugate was as shown in FIG. 2. See Table 30 for the related sequences of the antibody. Specifically, the heavy chain of the antibody comprised HCDR1 to HCDR3 having the amino acid sequences as shown in SEQ ID NOs: 1, 2 and 3, respectively, and the light chain of the antibody comprised LCDR1 to LCDR3 having the amino acid sequences as shown in SEQ ID NOs: 4, 5 and 6, respectively. The heavy chain of the antibody comprised a heavy chain variable region having the amino acid sequence as shown in SEQ ID NO: 7, and the light chain of the antibody comprised a light chain variable region having the amino acid sequence as shown in SEQ ID NO: 8.

The amino acid sequence of the transglutaminase used in this example was as follows:

DSDERVTPPAEPLDRMPDPYRPSYGRAETIVNNYIRKWQQVYSHRDGRKQQMTEEQREWLSYGCVG
VTWVNSGQYPTNRLAFAFFDEDKYKNELKNGRPRSGETRAEFEGRVAKDSFDEAKGFQRARDVASV
MNKALENAHDEGAYLDNLKKELANGNDALRNEDARSPFYSALRNTPSFKDRNGGNHDPSKMKAVI
YSKHFWSGQDRSGSSDKRKYGDPEAFRPDRGTGLVDMSRDRNIPRSPTSPGESFVNFDYGWFGAQTE
ADADKTVWTHGNHYHAPNGSLGAMHVYESKFRNWSDGYSDFDRGAYVVTFVPKSWNTAPDKVTQ
GWP (SEQ ID NO: 19)

### 1.2 Detection of ability of SYJS001-ADC of binding to CLDN18.2 protein by flow cytometry

In this experiment, the ability of SYJS001-ADC of binding to CLDN18.2 proteins of different species was detected by flow cytometry.

Cell lines that overexpress CLDN18.2 (HEK293-human CLDN18.2, HEK293-mouse CLDN18.2 and CHO-K1-cynomolgus monkey CLDN18.2) were incubated with SYJS001-ADC samples at different concentrations, and then incubated with a secondary antibody (goat anti-human IgG (H+L) cross-adsorbed secondary antibody) that binds to IgG. The fluorescence signal values at different concentrations were detected by a flow cytometer to analyze the abilities of the samples of binding to CLDN18.2 of different species.

### Specific experimental steps:

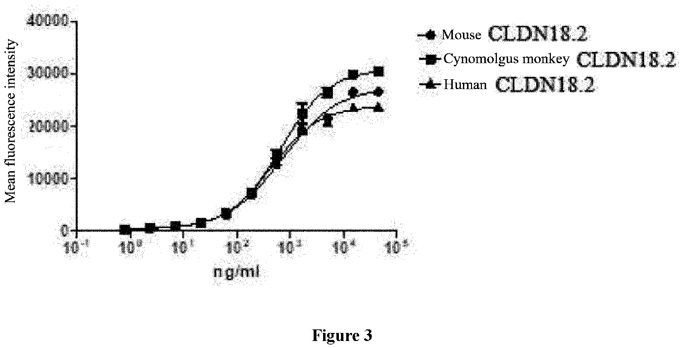

The protein sample SYJS001-ADC with the initial concentration of 45 µg/ml was subjected to 3-fold gradient dilution, with a total of 11 gradients. 100 µl of antibody diluents at different concentrations were taken and incubated with cells that express human, mouse and cynomolgus monkey CLDN18.2 (1 × 10⁶ cells/ml, 100 µl/well) at 4°C for 1.5 h, respectively. The cells were washed to remove unbound samples, the goat anti-human IgG (H+L) cross-adsorbed secondary antibody (1 : 1000 dilution) was added, and the cells were incubated at 4°C for 1 h. After washing, the fluorescence signal values of corresponding wells were detected by a flow cytometer. The data were analyzed using the GraphPad Prism 5 software. A regression model of the four-parameter equation was selected to plot an S-shaped curve, and the software automatically generated the median effective dose ED₅₀ (C value). The experimental results were as shown in FIG. 3 and Table 1, indicating that SYJS001-ADC has good affinity for human, mouse and cynomolgus monkey CLDN18.2.

**Table 1: ED₅₀ values for binding of SYJS001-ADC to CLDN18.2 of different species**

| **Sample names** | **ED₅₀ (ng/ml)** |
|---|---|
| HEK293-human CLDN18.2 | 439.1 |
| HEK293-mouse CLDN18.2 | 717.0 |
| CHO-K1-cynomolgus monkey CLDN18.2 | 647.9 |

### 1.3 Specific binding of SYJS001-ADC to CLDN18.2

HEK293-human CLDN18.1 cells and CHOK1-human CLDN18.2 cells were prepared according to a method similar to that in Example 1.2.

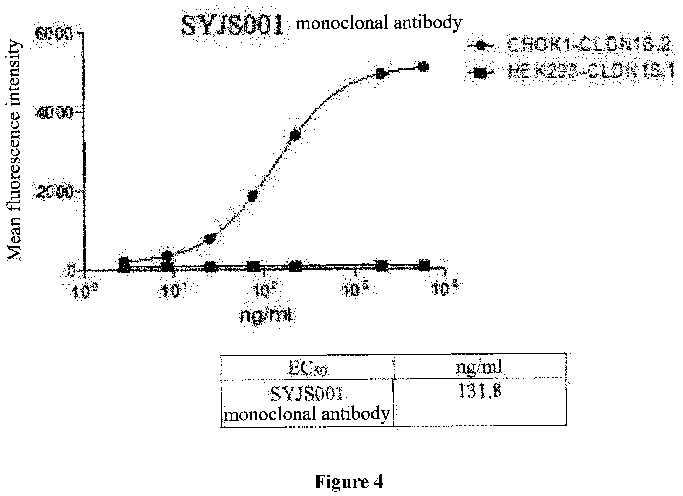

In the experiment of detecting the binding specificity of SYJS001 monoclonal antibody (FIG. 4), HEK293-human CLDN18.1 cells and CHOK1-human CLDN18.2 cells were cultured in corresponding complete media and passaged every 2-3 days. When the cell confluence reached 90%, the cell density was adjusted to 2-3 × 10⁶ cells/mL with a complete medium, and the cell suspension was plated into flow 96-well assay plates using a multi-channel pipette at 100 µL/well. Centrifugation was performed at 2500 rpm for 5 min, and the supernatant was discarded. The plates were washed twice with 2% FBS/PBS. The SYJS001 monoclonal antibody was diluted separately with 2% FBS/PBS buffer, 2 duplicate wells were set up for each concentration of the test substance, and corresponding blank controls were set up, wherein the monoclonal antibody and the blank controls were added at 100 µL/well, respectively. The plates were incubated at 4°C for 2 h, and washed three times with 2% FBS/PBS. 488-labeled goat anti-human IgG (1 : 1000 dilution) was added. The plates were incubated at 4°C for 1 h and washed three times with 2% FBS/PBS. The corresponding fluorescence channels were selected for reading.

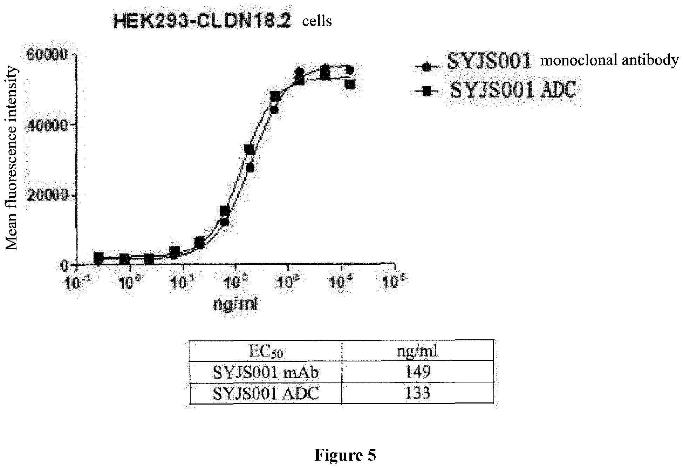

In the experiment of detecting the binding specificity of SYJS001 monoclonal antibody and SYJS001-ADC (FIG. 5), HEK293-human CLDN18.2 cells were cultured in a corresponding complete medium (Example 1.2) and passaged every 2-3 days. When the cell confluence reached 90%, the cell density was adjusted to 2-3 × 10⁶ cells/mL with a complete medium, and the cell suspension was plated into flow 96-well assay plates using a multi-channel pipette at 100 µL/well. Centrifugation was performed at 2500 rpm for 5 min, and the supernatant was discarded. The plates were washed twice with 2% FBS/PBS. The SYJS001 monoclonal antibody and the SYJS001-ADC were diluted with 2% FBS/PBS buffer, respectively. The ADC with the initial concentration of 15 µg/ml was subjected to 3-fold gradient dilution, with a total of 11 gradients. 2 duplicate wells were set up for each concentration of the test substance, and corresponding blank controls were set up, wherein the monoclonal antibody and the blank controls were added at 100 µL/well, respectively. The plates were incubated at 4°C for 2 h, and washed three times with 2% FBS/PBS. 488-labeled goat anti-human IgG (1 : 1000 dilution) was added. The plates were incubated at 4°C for 1 h and washed three times with 2% FBS/PBS. The corresponding fluorescence channels were selected for reading.

The results show (see FIG. 4 and FIG. 5) that the monoclonal antibody used in SYJS001 obtained in the present application can specifically bind to CLDN18.2, and has no obvious cross-reactivity to CLDN18.1; and compared to the naked antibody, SYJS001-ADC also specifically binds to CLDN18.2, and there is no obvious effect on the affinity after the conjugation to a toxin.

### 1.4 Evaluation of in-vivo efficacy of SYJS001-ADC

### 1.4.1 Comparison of efficacy with gemcitabine and cisplatin as controls

1) In this experiment, human pancreatic cancer Bxpc3-18.2 was selected to construct a nude mouse xenograft tumor model. When the tumor grew to a volume of about 100 mm³ (day 39 after inoculation), 48 animals with good tumor growth were selected and equally divided into 6 groups (day 0) according to the tumor volume, with 8 animals in each group. The animals were intravenously administered 0.9% sodium chloride injection (0.9 % INJ NS, solvent control group), SYJS001-ADC at 2, 4 and 8 mg/kg (single administration), SYJS001-mAb (SYJS001 naked antibody) at 8 mg/kg (single administration) and gemcitabine (GEM) at 50 mg/kg (biw×4, twice a week for a total of 4 weeks), respectively. The tumor diameters were measured twice a week, the mice were weighed, and the data were recorded. The tumor growth was dynamically observed by measuring the tumor diameters at different times after administration. The experiment was ended on day 28, the mice were asphyxiated with carbon dioxide, and then the tumors were stripped off and weighed.

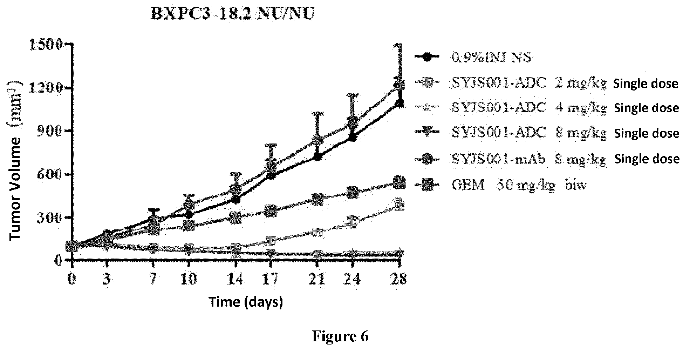

The results show that in this experiment, the tumor weight inhibition rates in the groups treated with SYJS001-ADC at 2, 4 and 8 mg/kg (single administration), the group treated with SYJS001-mAb at 8 mg/kg (single administration) and the group treated with Gemcitabine at 50 mg/kg (biw×4) are 56.6%, 94.8%, 97.8%, -36.2% and 51.0% respectively; compared with the solvent control group (0.9% sodium chloride injection), the tumor growth in the groups treated with SYJS001-ADC at 2, 4 and 8 mg/kg (single administration) and the group treated with GEM at 50 mg/kg (biw×4, twice a week, a total of four administrations) can be significantly inhibited (P < 0.01); and compared with the positive control group treated with GEM at 50 mg/kg (biw×4), the tumor inhibition effects in the groups treated with SYJS001-ADC at 4 and 8 mg/kg (single administration) are more significant, especially in the group treated with SYJS001-ADC at 8 mg/kg, with nearly 2 times (p < 0.001) the tumor inhibition effect (see FIG. 6).

2) In this experiment, human gastric cancer NUGC-4-18.2 was selected to construct a nude mouse xenograft tumor model. When the tumor grew to a volume of about 120 mm³ (day 6 after inoculation), 64 animals with good tumor growth were selected and equally divided into 8 groups (day 0) according to the tumor volume, with 8 animals in each group. The animals were intravenously administered 0.9% sodium chloride injection (0.9% INJ NS, solvent control group), SYJS001-ADC at 1, 2 and 4 mg/kg (qw×3), SYJS001-ADC at 4 and 8 mg/kg (single administration), SYJS001-mAb at 4 mg/kg (qw×3) and cisplatin at 6 mg/kg (qw×3, once a week for a total of 3 weeks), respectively. The tumor diameters were measured twice a week, the mice were weighed, and the data were recorded. The change in the tumor growth was dynamically observed by measuring the tumor diameters at different times after administration. The experiment was ended on day 20, the animals were asphyxiated with carbon dioxide, and then the tumors were stripped off and weighed.

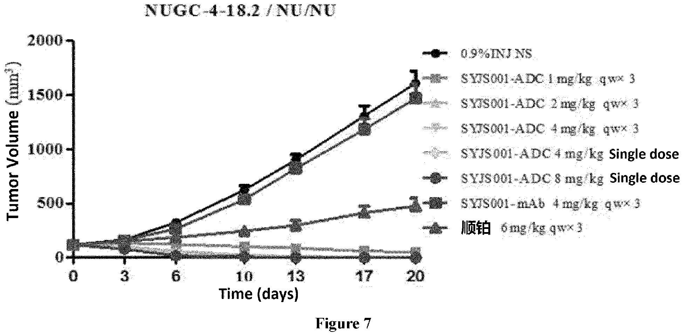

The results show that in this experiment, the tumor weight inhibition rates in the groups treated with SYJS001-ADC at 1, 2 and 4 mg/kg (qw×3), SYJS001-ADC at 4 and 8 mg/kg (single administration), SYJS001-mAb at 4 mg/kg (qw×3) and the group treated with cisplatin at 6 mg/kg (qw×3) are 98.0%, 100%, 100%, 100%, 100%, -1.2% and 66.5%, respectively; compared with the solvent control group, the tumor growth in all groups except for the group treated with SYJS001-mAb at 4 mg/kg (qw×3) can be significantly inhibited (p < 0.001); and the groups treated with SYJS001-ADC at 1, 2 and 4 mg/kg (qw×3) and the groups treated with SYJS001-ADC at 4 and 8 mg/kg (single administration) are significantly superior to the positive control group treated with cisplatin at 6 mg/kg (qw×3) (p < 0.05) in both terms of efficacy and toxicity (see FIG. 7).

### 1.4.2 Comparison of efficacy with IMAB362-ADC as control

IMAB362 (also known as claudiximab or zolbetuximab), a reported chimeric monoclonal antibody of the IgG1 subtype that selectively targets the first extracellular domain of CLDN18.2 and has little activity against CLDN18.1, was selected as a control antibody in this experiment.
1) In this experiment, human pancreatic cancer Bxpc3-18.2 was selected to construct a nude mouse xenograft tumor model. When the tumor grew to a volume of about 100 mm³ (day 39 after inoculation), 64 animals with good tumor growth were selected and equally divided into 8 groups (day 0) according to the tumor volume, with 8 animals in each group. The animals were intravenously administered 0.9% sodium chloride injection (0.9% INJ NS, solvent control group), SYJS001-ADC at 2, 4 and 8 mg/kg (single administration), IMAB362-ADC at 2, 4 and 8 mg/kg (single administration) (reference was made to patent CN 201680021997.6 for the IMAB362 sequence; gene synthesis was performed at Nanjing GenScript Biotechnology Co., Ltd.; protein expression was done by transient expression using a KOP293 transient transfection protein expression system from Zhuhai Kairui Biotechnology Co., Ltd.; and IMAB362-ADC was prepared with reference to the method for preparing SYJS001-ADC in Example 1.1) and SYJS001-mAb at 8 mg/kg (single administration), respectively. The tumor diameters were measured twice a week, the mice were weighed, and the data were recorded. The tumor growth was dynamically observed by measuring the tumor diameters at different times after administration. The experiment was ended on day 28, the mice were asphyxiated with carbon dioxide, and then the tumors were stripped off and weighed.

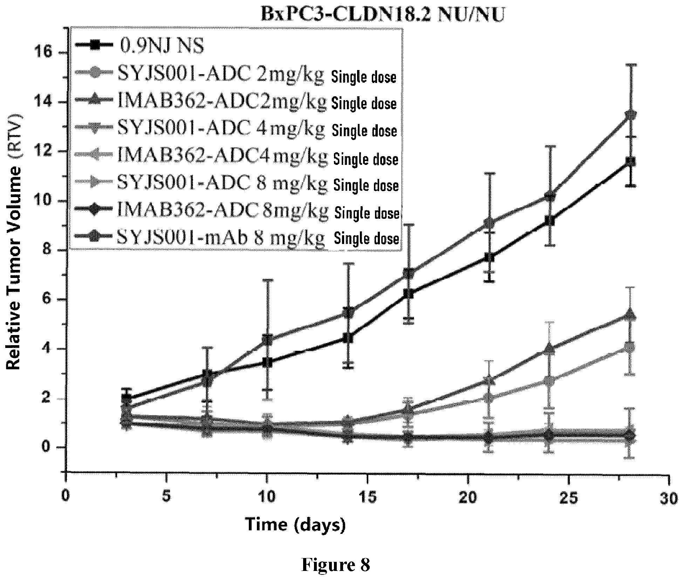

The results show that in this experiment, the tumor volume in the groups treated with SYJS001-ADC at 2, 4 and 8 mg/kg (single administration), the group treated with SYJS001-mAb at 8 mg/kg (single administration) and the groups treated with IMAB362-ADC at 2, 4 and 8 mg/kg (single administration) are 35.9%, 6.0%, 3.4%, 116.2%, 47.0%, 6.8% and 5.1% of the tumor volume in the solvent control group, respectively; compared with the solvent control group, the tumor growth in the groups treated with SYJS001-ADC at 2, 4 and 8 mg/kg (single administration) and the groups treated with IMAB362-ADC at 2, 4 and 8 mg/kg (single administration) can be significantly inhibited (p < 0.01); and compared with the positive control group treated with IMAB362-ADC, the effects are not much different at high doses, but at the low dose (effective dose), the in vivo tumor inhibition effect in the groups treated with SYJS001-ADC is significantly better than that in the groups treated with IMAB362-ADC. See FIG. 8 (RTV (relative tumor volume)) for details.

2) In this experiment, human gastric cancer NUGC-4-18.2 was selected to construct a nude mouse xenograft tumor model. When the tumor grew to a volume of about 120 mm³ (day 6 after inoculation), 64 animals with good tumor growth were selected and equally divided into 8 groups (day 0) according to the tumor volume, with 8 animals in each group. The animals were intravenously administered 0.9% sodium chloride injection (0.9% INJ NS, solvent control group), SYJS001-ADC at 0.5, 1, 2 and 4 mg/kg (single administration), IMAB362-ADC at 0.5, 1, 2 and 4 mg/kg (single administration) and SYJS001-mAb at 4 mg/kg (single administration), respectively. The tumor diameters were measured twice a week, the mice were weighed, and the data were recorded. The change in the tumor growth was dynamically observed by measuring the tumor diameters at different times after administration. The experiment was ended on day 20, the animals were asphyxiated with carbon dioxide, and then the tumors were stripped off and weighed.

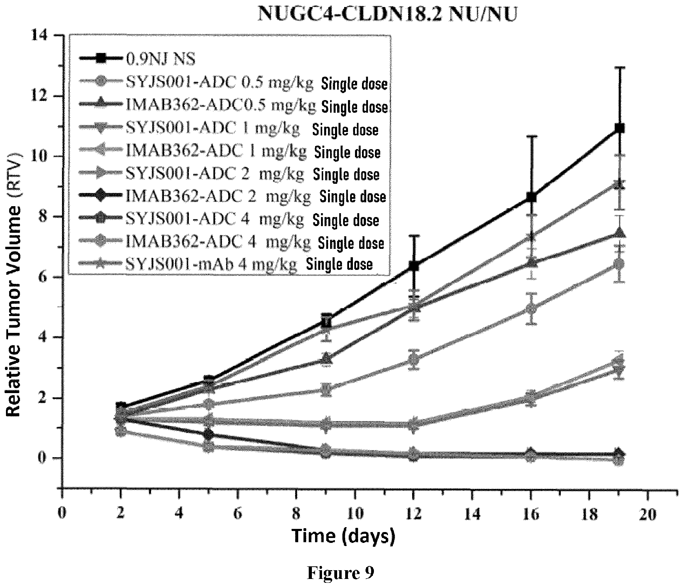

The results show that in this experiment, the tumor volumes in the groups treated with SYJS001-ADC at 0.5, 1, 2 and 4 mg/kg (single administration), the groups treated with IMAB362-ADC at 0.5, 1, 2 and 4 mg/kg (single administration) and the group treated with SYJS001-mAb at 4 mg/kg (single administration) are 59.1%, 27.3%, 1.8%, 0%, 68.2%, 30%, 1.8%, 0% and 83.6% of the tumor volume in the solvent control group, respectively; compared with the solvent control group, the tumor growth in all groups except for the group treated with SYJS001-mAb at 4 mg/kg can be significantly inhibited (p < 0.001); and the in vivo tumor inhibition effect on NUGC4-CLDN18.2 in the groups treated with SYJS001-ADC is not much different from that in the groups treated with IMAB362-ADC at high doses, but at the low dose (effective dose), the in vivo tumor inhibition effect in the groups treated with SYJS001-ADC is significantly superior to that in the groups treated with IMAB362-ADC (see FIG. 9).

### 1.5 Study on stability of ADC

The object of this experimental was to study the in vitro metabolic stability of SYJS001-ADC in plasma of different species (human, cynomolgus monkeys and SD rats). SYJS001-ADC was incubated at a concentration of 100 µg/mL under sterile conditions at 37°C for 0-168 h (7 days). The concentration of MMAE was detected by an LC-MS/MS method, and the concentrations of MMAE in 0.5% BSA-PBS, human plasma, cynomolgus monkey plasma and SD rat plasma were as shown in Table 2-Table 5. With the increase in the incubation time, free MMAE was generated in all substrates. After 168 h (7 days) of incubation at 37°C, SYJS001-ADC had shedding rates of 0.672%, 0.327%, 0.209% and 0.405% in 0.5% BSA-PBS, human plasma, cynomolgus monkey plasma and SD rat plasma, respectively. Experiments show that the shedding rates of MMAE on SYJS001-ADC are less than 1.0%, indicating that SYJS001-ADC is relatively stable in 0.5% BSA-PBS, human plasma, cynomolgus monkey plasma and SD rat plasma.

**Table 2: Concentration of free MMAE and shedding rate of MMAE on ADC in 0.5% BSA-PBS solution**

| | Concentration (ng/mL) | | | | | | Shedding rate (%) |
|---|---|---|---|---|---|---|---|
| | Time (h) | 1 | 2 | 3 | Mean | Standard deviation | |
| 0.5% BSA-PBS | 0 | 0.167 | 0.179 | 0.154 | 0.167 | 0.013 | 0.017 |
| | 8 | 0.662 | 0.635 | 0.682 | 0.660 | 0.024 | 0.069 |
| | 24 | 1.687 | 1.791 | 1.711 | 1.730 | 0.054 | 0.181 |
| | 48 | 2.808 | 2.732 | 2.871 | 2.804 | 0.070 | 0.293 |
| | 72 | 3.485 | 3.536 | 3.457 | 3.493 | 0.040 | 0.365 |
| | 96 | 4.532 | 4.446 | 4.238 | 4.405 | 0.151 | 0.460 |
| | 168 | 5.51 | 6.846 | 6.951 | 6.436 | 0.803 | 0.672 |

**Table 3: Concentration of free MMAE and shedding rate of MMAE on ADC in human plasma**

| | Concentration (ng/mL) | | | | | | Shedding rate (%) |
|---|---|---|---|---|---|---|---|
| | Time (h) | 1 | 2 | 3 | Mean | Standard deviation | |
| Human plasma | 0 | BQL | BQL | BQL | BQL | NA | NA |
| | 8 | 0.105 | 0.106 | 0.097 | 0.103 | 0.005 | 0.011 |
| | 24 | 0.39 | 0.33 | 0.31 | 0.343 | 0.042 | 0.036 |
| | 48 | 0.695 | 0.713 | 0.631 | 0.680 | 0.043 | 0.071 |
| | 72 | 1.05 | 1.045 | 0.932 | 1.009 | 0.067 | 0.105 |
| | 96 | 1.398 | 1.34 | 1.193 | 1.310 | 0.106 | 0.137 |
| | 168 | 3.017 | 3.122 | 3.238 | 3.126 | 0.111 | 0.327 |

**Table 4: Concentration of free MMAE and shedding rate of MMAE on ADC in cynomolgus monkey plasma**

| | Concentration (ng/mL) | | | | | | Shedding rate (%) |
|---|---|---|---|---|---|---|---|
| | Time (h) | 1 | 2 | 3 | Mean | Standard deviation | |
| Monkey plasma | 0 | BQL | BQL | BQL | BQL | NA | NA |
| | 8 | 0.118 | 0.137 | 0.138 | 0.131 | 0.011 | 0.014 |
| | 24 | 0.304 | 0.25 | 0.299 | 0.284 | 0.030 | 0.030 |
| | 48 | 0.549 | 0.556 | 0.543 | 0.549 | 0.007 | 0.057 |
| | 72 | 0.773 | 0.773 | 0.671 | 0.739 | 0.059 | 0.077 |
| | 96 | 1.042 | 0.971 | 0.978 | 0.997 | 0.039 | 0.104 |
| | 168 | 1.97 | 2.05 | 1.983 | 2.001 | 0.043 | 0.209 |

**Table 5: Concentration of free MMAE and shedding rate of MMAE on ADC in rat plasma**

| | Concentration (ng/mL) | | | | | | Shedding rate (%) |
|---|---|---|---|---|---|---|---|
| | Time (h) | 1 | 2 | 3 | Mean | Standard deviation | |
| Rat plasma | 0 | BQL | BQL | BQL | BQL | NA | NA |
| | 8 | 0.24 | 0.217 | 0.2 | 0.219 | 0.020 | 0.023 |
| | 24 | 0.565 | 0.537 | 0.532 | 0.545 | 0.018 | 0.057 |
| | 48 | 1.064 | 1.002 | 1.042 | 1.036 | 0.031 | 0.108 |
| | 72 | 1.498 | 1.393 | 1.538 | 1.476 | 0.075 | 0.154 |
| | 96 | 2.09 | 2.009 | 1.883 | 1.994 | 0.104 | 0.208 |
| | 168 | 4.041 | 3.801 | 3.793 | 3.878 | 0.141 | 0.405 |

In addition, the inventors also performed accelerated stability experiments. The specific steps were as follows: SYJS001-ADC was stored in a buffer (composed of w/v 5% L-histidine and 25% L-histidine hydrochloride) at 2°C to 8°C for different periods of time, and detected for stability. The results were as shown in the following table. The free MMAE of IMAB362-ADC (IMAB362-vcMMAE) reached 0.3% on day 28 (see CN 107667118A, Table 7), which was much higher than that (0.000026%) of the drug SYJS001-ADC of the present application by 3 months, indicating that the stability of SYJS001-ADC is much better than that of IMAB362-ADC.

**Table 6: Experimental results of accelerated stability of SYJS001-ADC**

| Investigation item | Time (months)-SYJS001-ADC | | | |
|---|---|---|---|---|
| | 0 month | 1 month | 2 months | 3 months |
| DAR distribution | DAR: 2.01 | 2.01 | 2.00 | 2.00 |
| | DAR2: 76.80% | 77.83% | 78.50% | 77.29% |
| Free MMAE | 0.000012% | 0.000018% | 0.000020% | 0.000026% |
| Free L&D (LND1002) | < 0.004% | < 0.004% | < 0.004% | < 0.004% |

| | | | | |
|---|---|---|---|---|
| Note: L&D is the abbreviation for linker-drug | | | | |

### 1.6 Preparation of pharmaceutical composition of SYJS001-ADC

Preparation steps of the pharmaceutical composition (taking the following formula as an example: 10 mg/ml SYJS001-ADC, 20 mmol/L histidine-histidine hydrochloride, 0.02% polysorbate 20 and 6% sucrose, pH 5.5):
(1) affinity chromatography: capturing the anti-CLDN18.2 antibody-drug conjugate SYJS001-ADC (a conjugate of an antibody and a drug, comprising one anti-CLDN18.2 antibody) that can be affinity-captured by Protein A by means of an affinity chromatography column (Mabselect sure chromatography medium); and allowing other components LND1002 and mTgase to directly flow through the column, washing the chromatography column to remove impurities, performing elution with a low-pH elution buffer, and collecting the eluate in a sterile container;
(2) liquid replacement using ultrafiltration: preparing a histidine-histidine hydrochloride solution with the same concentration as the formula amount of the final product, with a pH of 5.5, performing liquid replacement using a 30 kD ultrafiltration membrane pack to replace the affinity chromatography solution with the histidine-histidine hydrochloride solution, wherein the liquid replacement fold using ultrafiltration is no less than 100, the pH value of the replacement liquid is controlled to be consistent with that of the final product, while the concentration of the anti-CLDN18.2 antibody-drug conjugate SYJS001-ADC was not lower than 1.2 times the concentration of the finished product;
(3) preparation of semi-finished product diluent: according to the formula amount of the anti-CLDN18.2 antibody drug-conjugate SYJS001-ADC and the concentration of the anti-CLDN18.2 antibody-drug conjugate SYJS001-ADC obtained after liquid replacement using ultrafiltration, calculating the preparation amount of the final product, while calculating the required amount of various auxiliary materials and preparing a semi-finished product diluent; adding about 80% of water for injection into a preparation container, weighing histidine, histidine hydrochloride, polysorbate 20 and sucrose in sequence, adding the components into the preparation container, and stirring and mixing the components; and after the auxiliary materials in each group are completely dissolved, adding water for injection to adjust the volume to the preparation amount, stirring and mixing the resulting solution until uniform, and then filtering the resulting mixture for later use; and
(4) preparation of finished product: adding the prepared semi-finished product diluent to the prepared solution containing the anti-CLDN18.2 antibody-drug conjugate SYJS001-ADC liquid obtained after liquid replacement using ultrafiltration, using the diluent to adjust the volume to the preparation amount of the semi-finished product, and stirring and mixing the resulting solution until uniform; and performing sampling, detecting the protein content, the pH value and the microbial limits, performing sterile filtration, then subpackaging the products into sterilized injection bottles according to the specifications of the products, and plugging and capping the bottles.

### Example 2: Experiments of the effect of selection of pH values on stability of pharmaceutical preparation

pH value is an important factor that affects the stability of liquid preparations of biological products, has a regulatory effect on the charge distribution on the surface of proteins, affects the intermolecular and intramolecular forces of proteins, and affects the conformational stability, colloidal stability and chemical stability of proteins. In this experiment, 10 mg/ml SYJS001-ADC samples at different pH values (4.0, 4.5, 5.0, 5.5, 6.0, 6.5, 7.0, 7.5 and 8.0) were prepared with 20 mM citric acid-phosphate as a buffer system, and subpackaged in 2 ml vials, and the vials were sealed with rubber stoppers. The stability was studied at high temperature (40°C), and the thermal stability (DSC), physical stability (SEC-HPLC), chemical stability (CEX-HPLC) and the content of free toxins were evaluated. It can be seen from the experimental results that the stability is better in the pH range of 5.5 to 6.5.

### Detection methods:

DSC: The sample to be tested was diluted with the same buffer to a protein concentration of 1 mg/ml. 350 µl of the test sample and corresponding buffer were added into the corresponding positions in a 96-well plate, respectively. The 96-well plate was placed into a sample slot, and the temperature of the sample slot was set to 15°C. Instrument parameter settings: starting temperature 20°C, ending temperature 100°C, and heating rate 90°C/min.

SEC-HPLC: According to Pharmacopoeia of the People's Republic of China (edition 2020), volume IV, section 0512 high performance liquid chromatography, analysis and detection were performed using a chromatographic column with a chromatographic gel suitable for separating proteins with a molecular weight of 10 to 500 kD as a filler, and the proportions of the protein monomer peak, the high molecular weight species (HMWS) peak and the low molecular weight species (LMWS) peak were calculated according to the area normalization method.

CEX-HPLC: According to Pharmacopoeia of the People's Republic of China (edition 2020), volume IV, section 0512 high performance liquid chromatography, analysis and detection were performed using a cation exchange column as a chromatographic column, and the proportions of the acidic peak, main peak and basic peak of the test sample were calculated according to the area normalization method.

Content of free LND1002: According to Pharmacopoeia of the People's Republic of China (edition 2020), volume IV, section 0512 high performance liquid chromatography, a chromatographic column with octadecyl bonded silica gel as a filler was used, precipitation was performed with acetonitrile, the supernatant was taken for analysis and detection after ice bath, and the content of free LND1002 in the test sample was calculated by an external standard method.

The detection results were as follows:

**Table 7: DSC detection results**

| No. | pH value | Denaturation temperature (°C) | | |
|---|---|---|---|---|
| | | Tonset | Tm1 | Tm2 |
| 1 | 4.0 | 44.12 | 50.87 | 77.75 |
| 2 | 4.5 | 50.17 | 56.63 | 81.28 |
| 3 | 5.0 | 56.17 | 62.09 | 82.82 |
| 4 | 5.5 | 60.02 | 66.03 | 83.54 |
| 5 | 6.0 | 61.92 | 68.10 | 83.64 |
| 6 | 6.5 | 62.93 | 69.12 | 83.57 |
| 7 | 7.0 | 63.56 | 69.55 | 83.59 |
| 8 | 7.5 | 62.83 | 69.44 | 83.23 |
| 9 | 8.0 | 61.87 | 69.02 | 82.89 |

**Table 8: SEC-HPLC detection results (40°C for 2 weeks)**

| No. | pH value | Purity of SEC main peak (%) | | |
|---|---|---|---|---|
| | | 40°C-T0 | 40°C-7 d | 40°C-14 d |
| 1 | 4.0 | 99.16 | 98.17 | 96.43 |
| 2 | 4.5 | 99.16 | 98.08 | 98.14 |
| 3 | 5.0 | 99.15 | 98.85 | 98.27 |
| 4 | 5.5 | 99.16 | 99.10 | 98.18 |
| 5 | 6.0 | 99.10 | 99.01 | 98.10 |
| 6 | 6.5 | 99.08 | 98.88 | 97.94 |
| 7 | 7.0 | 99.05 | 98.77 | 97.67 |
| 8 | 7.5 | 99.00 | 98.57 | 97.46 |
| 9 | 8.0 | 98.97 | 98.51 | 97.21 |

**Table 9: CEX-HPLC detection results (40°C for 2 weeks)**

| No. | pH value | Purity of CEX main peak (%) | | |
|---|---|---|---|---|
| | | 40°C-T0 | 40°C-7 d | 40°C-14 d |
| 1 | 4.0 | 63.02 | 59.89 | 54.98 |
| 2 | 4.5 | 63.11 | 59.91 | 56.21 |
| 3 | 5.0 | 62.84 | 62.51 | 57.85 |
| 4 | 5.5 | 63.42 | 63.32 | 59.16 |
| 5 | 6.0 | 62.54 | 64.17 | 59.86 |
| 6 | 6.5 | 62.59 | 62.26 | 58.93 |
| 7 | 7.0 | 62.65 | 61.03 | 56.80 |
| 8 | 7.5 | 62.51 | 58.54 | 52.25 |
| 9 | 8.0 | 62.60 | 54.20 | 44.83 |

**Table 10: Free LND1002 detection results (40°C for 2 weeks)**

| No. | pH value | Content of free LND1002 (µg/ml) | | |
|---|---|---|---|---|
| | | 40°C-T0 | 40°C-7 d | 40°C-14 d |
| 1 | 4.0 | 0.103 | 0.311 | 0.325 |
| 2 | 4.5 | 0.080 | 0.288 | 0.320 |
| 3 | 5.0 | 0.050 | 0.308 | 0.294 |
| 4 | 5.5 | 0.042 | 0.296 | 0.376 |
| 5 | 6.0 | 0.026 | 0.311 | 0.388 |
| 6 | 6.5 | 0.011 | 0.265 | 0.331 |
| 7 | 7.0 | 0.009 | 0.223 | 0.237 |
| 8 | 7.5 | 0.006 | 0.202 | 0.218 |
| 9 | 8.0 | 0.007 | 0.131 | 0.170 |

### Example 3: Experiments of the effect of buffer on stability of pharmaceutical preparation

Buffer salts have important effects on maintaining the pH value, physical and chemical properties and stability of preparations. Buffer salts, such as citric acid-sodium citrate, succinic acid-sodium succinate and histidine-histidine hydrochloride, within the optimal pH range were selected as buffer components, with the screening range including, but not limited to, 10 to 40 mmol/L. The samples at different concentrations with different buffer salts, with the ADC concentration of 10 mg/ml, were prepared and subpackaged into injection bottles, and the stability was studied at high temperature (40°C). In the experiment, 10 mg/ml SYJS001 samples at pH 6.0 with three buffer systems (citric acid, succinic acid and histidine) having different ionic concentrations (10 mM, 20 mM and 40 mM) were prepared, and the thermal stability, physical stability, chemical stability and the content of free toxins were evaluated. The detection method was the same as that in Example 2. It can be seen from the experimental results that in the three buffer systems, thermal stability in succinic acid > thermal stability in citric acid > thermal stability in histidine, and the lower the salt concentration, the better the thermal stability; there is no significant difference in physical stability and chemical stability; and the content of free LND1002 with the two systems, i.e., succinic acid and histidine, is lower than that in citric acid. Preferably, the histidine and succinic acid buffer systems were used as basic buffers. and the concentration was preferably 20 mmol/L.

The specific detection results were as follows:

**Table 11: DSC detection results**

| No. | Buffer salt | Concentration (mmol/L) | Denaturation temperature (°C) | | |
|---|---|---|---|---|---|
| | | | Tonset | Tm1 | Tm2 |
| 1 | Citric acid-sodium citrate | 10 | 62.41 | 68.21 | 82.16 |
| 2 | | 20 | 61.73 | 67.33 | 83.20 |
| 3 | | 40 | 61.25 | 66.45 | 83.39 |
| 4 | Succinic acid-sodium succinate | 10 | 63.75 | 68.87 | 83.37 |
| 5 | | 20 | 63.17 | 68.04 | 83.35 |
| 6 | | 40 | 62.64 | 67.03 | 83.37 |
| 7 | Histidine-histidine hydrochloride | 10 | 61.57 | 68.15 | 83.00 |
| 8 | | 20 | 61.28 | 67.58 | 82.90 |
| 9 | | 40 | 60.70 | 66.81 | 82.71 |

**Table 12: SEC-HPLC detection results (40°C for 2 weeks)**

| No. | Buffer salt | Concentration (mmol/L) | Purity of SEC main peak (%) | | |
|---|---|---|---|---|---|
| | | | 40°C-T0 | 40°C-7 d | 40°C-14 d |
| 1 | Citric acid-sodium citrate | 10 | 98.90 | 99.35 | 98.73 |
| 2 | | 20 | 98.96 | 99.33 | 98.75 |
| 3 | | 40 | 98.91 | 99.34 | 98.75 |
| 4 | Succinic acid-sodium succinate | 10 | 98.76 | 99.36 | 98.73 |
| 5 | | 20 | 98.85 | 99.35 | 98.75 |
| 6 | | 40 | 98.88 | 99.34 | 98.77 |
| 7 | Histidine-histidine hydrochloride | 10 | 98.83 | 99.47 | 98.85 |
| 8 | | 20 | 98.87 | 99.47 | 98.91 |
| 9 | | 40 | 98.96 | 99.53 | 99.00 |

**Table 13: CEX-HPLC detection results (40°C for 2 weeks)**

| No. | Buffer salt | Concentration (mmol/L) | Purity of CEX main peak (%) | | |
|---|---|---|---|---|---|
| | | | 40°C-T0 | 40°C-7 d | 40°C-14 d |
| 1 | Citric acid-sodium citrate | 10 | 60.01 | 59.40 | 57.50 |
| 2 | | 20 | 59.24 | 57.94 | 58.31 |
| 3 | | 40 | 60.26 | 59.32 | 58.37 |
| 4 | Succinic acid-sodium succinate | 10 | 61.20 | 59.87 | 57.75 |
| 5 | | 20 | 61.27 | 59.81 | 57.80 |
| 6 | | 40 | 60.23 | 59.16 | 57.80 |
| 7 | Histidine-histidine hydrochloride | 10 | 60.78 | 59.95 | 58.18 |
| 8 | | 20 | 61.50 | 59.89 | 58.58 |
| 9 | | 40 | 60.86 | 60.12 | 58.67 |

**Table 14: Free LND1002 detection results (40°C for 2 weeks)**

| No. | Buffer salt | Concentration (mmol/L) | Content of free LND1002 (µg/ml) | | |
|---|---|---|---|---|---|
| | | | 40°C-T0 | 40°C-7 d | 40°C-14 d |
| 1 | Citric acid-sodium citrate | 10 | 0.011 | 0.069 | 0.235 |
| 2 | | 20 | 0.012 | 0.042 | 0.203 |
| 3 | | 40 | 0.013 | 0.046 | 0.160 |
| 4 | Succinic acid-sodium succinate | 10 | 0.013 | 0.077 | 0.118 |
| 5 | | 20 | 0.012 | 0.098 | 0.116 |
| 6 | | 40 | 0.011 | 0.072 | 0.141 |
| 7 | Histidine-histidine hydrochloride | 10 | 0.025 | 0.070 | 0.119 |
| 8 | | 20 | 0.021 | 0.061 | 0.124 |
| 9 | | 40 | 0.036 | 0.081 | 0.142 |

### Example 4: Experiments of the effects of selections of surfactant and stabilizer on stability of pharmaceutical composition

On the basis of the preferred pH value (6.0) and buffer salt (histidine-histidine hydrochloride), polysorbate 20, polysorbate 80, sucrose, trehalose, mannitol and sorbitol were added, and the ADC concentration was 10 mg/ml. The samples were subpackaged into injection bottles, the stability was studied at high temperature (40°C), and the thermal stability, physical stability and chemical stability were evaluated. The detection method was the same as that in Example 2. It can be seen from the experimental results that the thermal stability of the product can be improved by the addition of the stabilizer, and there is no significant change in the molecular size heterogeneities and charge heterogeneities of the samples in each group under the existing treatment conditions, indicating that the stability of the product is good. Preferably, sugar was used as a stabilizer on the basis of the addition of the surfactant. The specific detection results were as follows:

**Table 15: DSC detection results**

| No. | Buffer salt | Surfactant | Stabilizer | Denaturation temperature (°C) | | |
|---|---|---|---|---|---|---|
| | | | | Tonset | Tm1 | Tm2 |
| 1 | | / | / | 61.28 | 69.02 | 83.10 |
| 2 | | Polysorbate 20 (0.02%) | / | 62.02 | 68.68 | 83.10 |
| 3 | | Polysorbate 80 (0.02%) | / | 62.03 | 68.73 | 83.12 |
| 4 | | / | Sucrose (6%) | 62.95 | 69.60 | 83.72 |
| 5 | | / | Trehalose (6%) | 62.72 | 69.75 | 83.76 |
| 6 | | / | Mannitol (3%) | 62.41 | 69.32 | 83.52 |
| 7 | 20 mmol/L Histidine-histidine hydrochloride pH 6.0 | / | Sorbitol (3%) | 62.68 | 69.32 | 83.55 |
| 8 | | Polysorbate 20 (0.02%) | Sucrose (6%) | 62.40 | 69.75 | 83.66 |
| 9 | | Polysorbate 20 (0.02%) | Trehalose (6%) | 62.97 | 69.70 | 83.73 |
| 10 | | Polysorbate 20 (0.02%) | Mannitol (3%) | 61.81 | 69.44 | 83.47 |
| 11 | | Polysorbate 20 (0.02%) | Sorbitol (3%) | 62.13 | 69.37 | 83.49 |
| 12 | | Polysorbate 80 (0.02%) | Sucrose (6%) | 63.50 | 69.36 | 83.73 |
| 13 | | Polysorbate 80 (0.02%) | Trehalose (6%) | 63.42 | 69.37 | 83.78 |
| 14 | | Polysorbate 80 (0.02%) | Mannitol (3%) | 62.25 | 69.43 | 83.49 |
| 15 | | Polysorbate 80 (0.02%) | Sorbitol (3%) | 62.58 | 69.34 | 83.52 |

**Table 16: SEC-HPLC detection results (40°C for 4 weeks)**

| No. | Buffer salt | Surfactant | Stabilizer | Purity of SEC main peak (%) | | |
|---|---|---|---|---|---|---|
| | | | | 40°C-T0 | 40°C-14 d | 40°C-28 d |
| 1 | | / | / | 99.04 | 97.59 | 97.21 |
| 2 | | Polysorbate 20 (0.02%) | / | 99.05 | 97.53 | 97.02 |
| 3 | | Polysorbate 80 (0.02%) | / | 99.05 | 97.48 | 97.03 |
| 4 | | / | Sucrose (6%) | 99.10 | 97.71 | 97.30 |
| 5 | | / | Trehalose (6%) | 99.07 | 97.71 | 97.30 |
| 6 | | / | Mannitol (3%) | 98.50 | 97.65 | 97.30 |
| 7 | 20 mmol/L Histidine-histidine hydrochloride pH 6.0 | / | Sorbitol (3%) | 99.19 | 97.63 | 97.36 |
| 8 | | Polysorbate 20 (0.02%) | Sucrose (6%) | 99.08 | 97.62 | 97.14 |
| 9 | | Polysorbate 20 (0.02%) | Trehalose (6%) | 99.04 | 97.57 | 97.08 |
| 10 | | Polysorbate 20 (0.02%) | Mannitol (3%) | 99.04 | 97.53 | 97.08 |
| 11 | | Polysorbate 20 (0.02%) | Sorbitol (3%) | 99.06 | 97.62 | 97.13 |
| 12 | | Polysorbate 80 (0.02%) | Sucrose (6%) | 99.05 | 97.57 | 97.20 |
| 13 | | Polysorbate 80 (0.02%) | Trehalose (6%) | 99.03 | 97.55 | 97.12 |
| 14 | | Polysorbate 80 (0.02%) | Mannitol (3%) | 99.04 | 97.55 | 97.12 |
| 15 | | Polysorbate 80 (0.02%) | Sorbitol (3%) | 99.06 | 97.53 | 97.14 |

**Table 17: CEX-HPLC detection results (40°C for 4 weeks)**

| No. | Buffer salt | Surfactant | Stabilizer | Purity of CEX main peak (%) | | |
|---|---|---|---|---|---|---|
| | | | | 40°C-T0 | 40°C-14 d | 40°C-28 d |
| 1 | | / | / | 60.20 | 59.59 | 53.61 |
| 2 | | Polysorbate 20 (0.02%) | / | 59.78 | 60.57 | 53.35 |
| 3 | | Polysorbate 80 (0.02%) | / | 60.60 | 61.02 | 54.17 |
| 4 | | / | Sucrose (6%) | 60.00 | 60.32 | 54.89 |
| 5 | | / | Trehalose (6%) | 60.29 | 60.63 | 55.02 |
| 6 | | / | Mannitol (3%) | 61.82 | 60.23 | 54.91 |
| 7 | | / | Sorbitol (3%) | 61.70 | 60.69 | 53.24 |
| 8 | 20 mmol/L Histidine-histidine hydrochloride pH 6.0 | Polysorbate 20 (0.02%) | Sucrose (6%) | 60.13 | 60.29 | 53.50 |
| 9 | | Polysorbate 20 (0.02%) | Trehalose (6%) | 59.79 | 60.11 | 53.49 |
| 10 | | Polysorbate 20 (0.02%) | Mannitol (3%) | 59.87 | 60.04 | 53.60 |
| 11 | | Polysorbate 20 (0.02%) | Sorbitol (3%) | 60.33 | 59.59 | 53.00 |
| 12 | | Polysorbate 80 (0.02%) | Sucrose (6%) | 59.87 | 59.99 | 53.58 |
| 13 | | Polysorbate 80 (0.02%) | Trehalose (6%) | 60.04 | 59.73 | 53.67 |
| 14 | | Polysorbate 80 (0.02%) | Mannitol (3%) | 59.97 | 59.38 | 53.92 |
| 15 | | Polysorbate 80 (0.02%) | Sorbitol (3%) | 60.12 | 60.15 | 53.15 |

### Example 5: Formula optimization confirmation experiment

On the basis of the results of the previous formula experiments, the interaction among various factors including the pH values, the buffer salts, the surfactants and the stabilizers was comprehensively considered, wherein the pH values include 5.5, 6.0 and 6.5; the buffer salts include succinic acid and histidine, with a concentration of 20 mmol/L; the surfactants include polysorbate 20 and polysorbate 80, with a concentration of 0.02%; and the stabilizers include sucrose and trehalose, with a concentration of 6%. In the experiment, a total of 12 groups of formulas were designed, and the ADC concentration was 10 mg/ml. The samples in each group were subpackaged into injection bottles, the stability was studied under the conditions of high temperature (45°C) and illumination (45001x ± 500lx), and the thermal stability, physical stability, chemical stability and the content of free toxins were evaluated. The detection method was the same as that in Example 2, and the experimental results were comprehensively analyzed, with the Tm2 and the changes in the purity of SEC monomer, the purity of CEX main peak and the content of free toxins at the end of experiments under illumination and high temperature of each formula as determination conditions. The results show that the pH values and the type of the buffer salts have a relatively high effect on the product quality, the surfactants and the stabilizers have no significant effect on the product quality, but the overall analysis shows that the degradation rate of each combined formula is not significant under strong destruction conditions, indicating that the products under the above-mentioned combined formulas have good stability. According to the comprehensive analysis of the experiments, on the basis that the pH values and the buffer salts had relatively high effect on the product, the combination of 10 mg/ml SYJS001-ADC, 20 mmol/L histidine, 0.02% polysorbate 20 and 6% sucrose and pH 5.5, was preferably used as the formula for preparing a clinical sample.

**Table 18: Experimental design of the composition of 12 groups**

| No. | pH value | Buffer salt (20 mmol/L) | Surfactant (0.02%) | Stabilizer (6%) |
|---|---|---|---|---|
| 1 | 5.5 | Histidine | Polysorbate 20 | Trehalose |
| 2 | 5.5 | Histidine | Polysorbate 80 | Sucrose |
| 3 | 6.0 | Histidine | Polysorbate 20 | Trehalose |
| 4 | 6.0 | Histidine | Polysorbate 80 | Sucrose |
| 5 | 6.5 | Histidine | Polysorbate 20 | Trehalose |
| 6 | 6.5 | Histidine | Polysorbate 80 | Sucrose |
| 7 | 5.5 | Succinic acid | Polysorbate 20 | Sucrose |
| 8 | 5.5 | Succinic acid | Polysorbate 80 | Trehalose |
| 9 | 6.0 | Succinic acid | Polysorbate 20 | Sucrose |
| 10 | 6.0 | Succinic acid | Polysorbate 80 | Trehalose |
| 11 | 6.5 | Succinic acid | Polysorbate 20 | Sucrose |
| 12 | 6.5 | Succinic acid | Polysorbate 80 | Trehalose |

**Table 19: DSC detection results**

| No. | Name | Denaturation temperature (°C) | | |
|---|---|---|---|---|
| | | Tonset | Tm1 | Tm2 |
| 1 | 5.5-H-PS80-Suc | 57.19 | 63.42 | 80.26 |
| 2 | 5.5-H-PS20-Tre | 58.40 | 63.38 | 80.78 |
| 3 | 6.0-H-PS80-Suc | 60.97 | 67.05 | 82.04 |
| 4 | 6.0-H-PS20-Tre | 62.11 | 67.04 | 82.15 |
| 5 | 6.5-H-PS80-Suc | 63.26 | 69.94 | 82.53 |
| 6 | 6.5-H-PS20-Tre | 63.43 | 69.83 | 82.55 |
| 7 | 5.5-S-PS80-Tre | 60.76 | 66.78 | 82.44 |
| 8 | 5.5-S-PS20-Suc | 59.51 | 66.58 | 82.15 |
| 9 | 6.0-S-PS80-Tre | 62.70 | 68.23 | 82.54 |
| 10 | 6.0-S-PS20-Suc | 62.52 | 67.95 | 82.36 |
| 11 | 6.5-S-PS80-Tre | 63.43 | 69.05 | 82.15 |
| 12 | 6.5-S-PS20-Suc | 63.30 | 68.78 | 81.83 |

Remarks: Name consists of pH, buffer salt, surfactant and stabilizer, where H represents histidine, S represents succinic acid, PS represents polysorbate, Suc represents sucrose, and Tre represents trehalose.

**Table 20: SEC-HPLC detection results (45°C for 2 weeks, illumination for 10 days)**

| No. | Name | Purity of main peak at high temperature (%) | | | Purity of main peak under illumination (%) | | |
|---|---|---|---|---|---|---|---|
| | | 0 d | 7d | 14 d | 0 d | 5 d | 10 d |
| 1 | 5.5-H-PS80-Suc | 98.97 | 99.03 | 99.23 | 98.97 | 98.98 | 99.15 |
| 2 | 5.5-H-PS20-Tre | 98.94 | 98.95 | 99.20 | 98.94 | 98.85 | 99.03 |
| 3 | 6.0-H-PS80-Suc | 98.90 | 98.97 | 99.02 | 98.90 | 98.80 | 99.01 |
| 4 | 6.0-H-PS20-Tre | 98.86 | 98.92 | 98.92 | 98.86 | 98.70 | 98.73 |
| 5 | 6.5-H-PS80-Suc | 98.85 | 98.91 | 98.96 | 98.85 | 98.69 | 98.75 |
| 6 | 6.5-H-PS20-Tre | 98.84 | 98.91 | 98.86 | 98.84 | 98.67 | 98.69 |
| 7 | 5.5-S-PS80-Tre | 98.84 | 98.90 | 98.90 | 98.84 | 98.30 | 98.04 |
| 8 | 5.5-S-PS20-Suc | 98.85 | 98.84 | 98.86 | 98.85 | 98.53 | 98.25 |
| 9 | 6.0-S-PS80-Tre | 98.79 | 98.84 | 98.85 | 98.79 | 98.18 | 97.78 |
| 10 | 6.0-S-PS20-Suc | 98.83 | 98.71 | 98.82 | 98.83 | 97.99 | 97.42 |
| 11 | 6.5-S-PS80-Tre | 98.78 | 98.75 | 98.67 | 98.78 | 96.52 | 95.99 |
| 12 | 6.5-S-PS20-Suc | 98.78 | 98.91 | 96.05 | 98.78 | 97.23 | 96.49 |

**Table 21: CEX-HPLC detection results (45°C for 2 weeks, illumination for 10 days)**

| No. | Name | Purity of main peak at high temperature (%) | | | Purity of main peak under illumination (%) | | |
|---|---|---|---|---|---|---|---|
| | | 0 d | 7d | 14 d | 0 d | 5 d | 10 d |
| 1 | 5.5-H-PS80-Suc | 55.23 | 53.19 | 50.26 | 55.23 | 53.48 | 53.86 |
| 2 | 5.5-H-PS20-Tre | 54.20 | 54.38 | 50.15 | 54.20 | 52.67 | 54.72 |
| 3 | 6.0-H-PS80-Suc | 54.70 | 54.39 | 49.41 | 54.70 | 53.13 | 51.73 |
| 4 | 6.0-H-PS20-Tre | 54.53 | 54.25 | 49.29 | 54.53 | 52.90 | 51.72 |
| 5 | 6.5-H-PS80-Suc | 55.27 | 54.26 | 49.08 | 55.27 | 52.13 | 50.88 |
| 6 | 6.5-H-PS20-Tre | 54.81 | 53.75 | 49.19 | 54.81 | 52.59 | 50.37 |
| 7 | 5.5-S-PS80-Tre | 54.64 | 54.48 | 49.77 | 54.64 | 54.37 | 51.89 |
| 8 | 5.5-S-PS20-Suc | 54.53 | 53.45 | 48.92 | 54.53 | 52.91 | 51.15 |
| 9 | 6.0-S-PS80-Tre | 54.14 | 53.45 | 48.78 | 54.14 | 52.14 | 50.86 |
| 10 | 6.0-S-PS20-Suc | 53.77 | 52.80 | 49.48 | 53.77 | 54.95 | 53.16 |
| 11 | 6.5-S-PS80-Tre | 54.64 | 53.01 | 48.75 | 54.64 | 53.47 | 50.10 |
| 12 | 6.5-S-PS20-Suc | 53.92 | 53.91 | 48.02 | 53.92 | 52.22 | 52.46 |

**Table 22: Free LND1002 detection results (45°C for 2 weeks, illumination for 10 days)**

| No. | Name | Content of free LND1002 at high temperature (µg/ml) | | | Content of free LND1002 under illumination (µg/ml) | | |
|---|---|---|---|---|---|---|---|
| | | 0 d | 7d | 14 d | 0 d | 5 d | 10d |
| 1 | 5.5-H-PS80-Suc | 0.0246 | 0.0563 | 0.1400 | 0.0246 | 0.0237 | 0.0220 |
| 2 | 5.5-H-PS20-Tre | 0.0237 | 0.1070 | 0.1443 | 0.0237 | 0.0253 | 0.0290 |
| 3 | 6.0-H-PS80-Suc | 0.0097 | 0.1162 | 0.2019 | 0.0097 | 0.0200 | 0.0481 |
| 4 | 6.0-H-PS20-Tre | 0.0152 | 0.1204 | 0.2017 | 0.0152 | 0.0210 | 0.0438 |
| 5 | 6.5-H-PS80-Suc | 0.0050 | 0.0793 | 0.1531 | 0.0050 | 0.0107 | 0.0420 |
| 6 | 6.5-H-PS20-Tre | 0.0073 | 0.0931 | 0.1918 | 0.0073 | 0.0163 | 0.0241 |
| 7 | 5.5-S-PS80-Tre | 0.0253 | 0.1505 | 0.2202 | 0.0253 | 0.0220 | 0.0764 |
| 8 | 5.5-S-PS20-Suc | 0.0188 | 0.1803 | 0.2280 | 0.0188 | 0.0171 | 0.0841 |
| 9 | 6.0-S-PS80-Tre | 0.0156 | 0.1654 | 0.2398 | 0.0156 | 0.0125 | 0.0828 |
| 10 | 6.0-S-PS20-Suc | 0.0101 | 0.1220 | 0.2133 | 0.0101 | 0.0149 | 0.0564 |
| 11 | 6.5-S-PS80-Tre | 0.0093 | 0.1443 | 0.2016 | 0.0093 | 0.0133 | - |
| 12 | 6.5-S-PS20-Suc | 0.0107 | 0.1437 | 0.2043 | 0.0107 | 0.0183 | 0.1006 |

| | | | | | | | |
|---|---|---|---|---|---|---|---|
| Note: "-" means no results detected, free LND1002 detection results are below the methodological limit of quantitation (0.4 µg/ml), for reference only. | | | | | | | |

### Example 6: Study on stability of formula

### 6.1 Study on stability at high temperature and under illumination

A group of samples were prepared according to the confirmed formula: 10 mg/ml SYJS001-ADC, 20 mmol/L histidine, 0.02% polysorbate 20 and 6% sucrose and pH 5.5, and the stability of the samples was preliminarily studied. See the following table for the treatment conditions and detection items:

**Table 23: Study on stability of preferred preparation formula at high temperature and under illumination**

| Detection items | T0 | High temperature (37°C ± 2°C) | | Illumination (4500lx) | |
|---|---|---|---|---|---|
| | | 5 d | 10 d | 5 d | 10 d |
| SEC-HPLC (monomer, %) | 99.24 | 99.21 | 99.10 | 99.21 | 99.12 |
| CEX-HPLC (main peak, %) | 55.30 | 54.73 | 53.48 | 54.03 | 53.84 |
| NR-CE-SDS (monomer, %) | 97.15 | 96.60 | 97.00 | 96.82 | 97.36 |
| R-CE-SDS (LC+HC, %) | 98.35 | 98.48 | 98.42 | 98.55 | 98.57 |
| DAR value | 2.01 | 2.01 | 2.01 | 2.01 | 2.01 |
| Amount of unconjugated antibody (%) | 0.40 | 0.39 | 0.40 | 0.31 | 0.34 |
| Content of free LND1002 (µg/ml) | 0.0206 | 0.0546 | 0.0691 | 0.0161 | 0.0141 |

The above-mentioned research results show that the pharmaceutical preparation of the present application is not significantly degraded under the conditions of high temperature, indicating that the quality of the formula is stable and reliable.

### 6.2 Study on long-term stability

The optimized formula was composed of: 10 mg/ml SYJS001-ADC, 20 mmol/L histidine-histidine hydrochloride, 0.02% polysorbate 20, 6% sucrose and the balance being water for injection, with a pH of 5.5. Three batches of samples were prepared under GMP conditions. The stability of the three batches of samples was studied. The methods for detecting various research indicators were all verified by methodology, and were accurate and reliable. The stability of the three batches of samples at both - 20°C ± 5°C and 5°C ± 3°C was studied. In addition, the long-term stability of the injection at both - 20°C ± 5°C and 5°C ± 3°C was further studied.

### Detection methods:

SEC-HPLC: According to Pharmacopoeia of the People's Republic of China (edition 2020), volume IV, section 0512 high performance liquid chromatography, a chromatographic column with a chromatographic gel suitable for separating proteins with a molecular weight of 10 to 500 kD as a filler (such as XBridge^{®}BEH200Å, 7.8 × 300 mm, 200 Å, 3.5 µm or other suitable chromatographic columns) was used, with mobile phase: 100 mmol/L phosphate buffer, 100 mmol/L NaCl and 10% isopropanol, with a pH of 6.7 ± 0.1; flow rate: 0.8 ml/min; detection wavelength: 280 nm. 30 µg ± 3 µg of test sample solution was taken and injected into a liquid chromatograph, and the proportions of the protein monomer peak, the high molecular weight species (HMWS) peak and the low molecular weight species (LMWS) peak were calculated according to the area normalization method.

CEX-HPLC: According to Pharmacopoeia of the People's Republic of China (edition 2020), volume IV, section 0512 high performance liquid chromatography, an ion exchange column (such as MAbPac^{™} SCX-10, 4 × 250 mm or other suitable chromatographic columns) was used as the chromatographic column, with mobile phase A: 20 mmol/L CAPSO and 20 mmol/L NaCl, with a pH of 8.0; mobile phase B: 20 mmol/L CAPSO and 300 mmol/L NaCl, with a pH of 10.0; flow rate: 1.0 ml/min; detection wavelength: 280 nm. 40 µg ± 4 µg of test sample solution was taken and injected into a liquid chromatograph for gradient elution, and the proportions of the acidic peak, the main peak and the basic peak of the test sample were calculated according to the area normalization method.

R-CE-SDS: According to Pharmacopoeia of the People's Republic of China (edition 2020), volume IV, section 3127 determination of molecular size heterogeneities in monoclonal antibodies, the proportions of the heavy chain peak, the light chain peak and the non-glycosylated heavy chain peak were calculated according to the area normalization method.

NR-CE-SDS: According to Pharmacopoeia of the People's Republic of China (edition 2020), volume IV, section 3127 determination of molecular size heterogeneities in monoclonal antibodies, the proportions of the monomer peak and the low molecular weight species (2HC1LC and 1HC1LC) peak were calculated according to the area normalization method.

DAR distribution: According to Pharmacopoeia of the People's Republic of China (edition 2020), volume IV, section 0512 high performance liquid chromatography, a chromatographic column with tetraalkyl bonded silica gel as a filler (such as PLRP-S 1000A, 2.1 × 50 mm, particle size 5 µm or other suitable chromatographic columns) was used, with mobile phase A: 0.1% (v/v) aqueous TFA; mobile phase B: 0.1% (v/v) TFA acetonitrile solution; column temperature: 80°C; flow rate: 0.25 ml/min; detection wavelength: 280 nm. 10 µL (10 µg ± 1 µg) of test sample solution was taken and injected into a liquid chromatograph for gradient elution, and the DAR value and the DAR2 content were calculated.

Amount of unconjugated antibody: With the same method as the method for detecting DAR distribution, analysis was performed by an external standard method using a spiked control, and the content of the unconjugated antibody was calculated according to the external standard method.

Content of free LND1002: According to Pharmacopoeia of the People's Republic of China (edition 2020), volume IV, section 0512 high performance liquid chromatography, a chromatographic column with octadecyl bonded silica gel as a filler (such as Agilent Poroshell 120 SB-C18, 4.6 × 150 mm, particle size 2.7 µm or other suitable chromatographic columns) was used, with mobile phase A: 0.1% aqueous TFA; mobile phase B: 0.1% TFA in acetonitrile; column temperature: 30°C; flow rate: 0.5 ml/min; detection wavelength: 250 nm. 100 µL (10 mg/mL) of test sample was taken and precipitated with acetonitrile at 1 : 3. The resulting mixture was placed in an ice bath at - 20°C for 60 min and then centrifuged. The supernatant was taken for sample loading, gradient elution was performed using a certain amount of LND1002 (loading amount of 65 ng) as the external standard control, and the content of free LND1002 in the test sample was calculated according to the external standard method. Content of free LND002 (%) = Detected concentration of LND1002/ADC concentration × 100%

Content of free MMAE: According to Pharmacopoeia of the People's Republic of China (edition 2020), volume IV, section 0512 high performance liquid chromatography and section 0431 mass spectrometry, a chromatographic column with octadecyl bonded silica gel as a filler (such as Waters ACQUITY UPLC^{®} BEH-C18, 2.1 × 50 mm, particle size 1.7 µm or other suitable chromatographic columns) was used, with mobile phase A: 0.1% aqueous TFA; mobile phase B: 0.1% TFA in acetonitrile; column temperature: 40°C; flow rate: 0.4 ml/min; detection wavelength: 250 nm. 50 µL (10 mg/mL) of test sample was taken, an internal standard working liquid was added, and the resulting mixture was precipitated with acetonitrile at 1 : 3, placed in an ice bath at - 20°C for 60 min, and then centrifuged. The supernatant was taken for sample loading, and MMAE at different concentrations were loaded as the controls. The samples were subjected to gradient elution by liquid chromatography and then entered a mass spectrometer. The parameters such as capillary and cone voltages, ion source temperature, collision energy and atomization flow rate of the mass spectrometer were set, analysis was then performed, and the content of free MMAE in the test sample was calculated according to a standard curve. Content of free MMAE (%) = Detected concentration of MMAE/ADC concentration × 100%

Antigen binding activity: determined according to enzyme-linked immunoassay (ELISA).

Biological activity: determined according to cell proliferation inhibition assay.

### Detection results:

**Table 24: Stability results of key quality indicators of batch 1 under GMP**

| Investigation item | Batch 1 | | | |
|---|---|---|---|---|
| | 0 month | - 20°C ± 5°C | | |
| | | 12 months | 24 months | 30 months |
| SEC-HPLC | HMWS: 0.9% | 0.7% | 1.0% | 1.0% |
| | Monomer: 98.4% | 98.5% | 98.4% | 98.2% |
| | LMWS: 0.7% | 0.9% | 0.6% | 0.9% |
| CEX-HPLC | Acid peak: 12.4% | 13.0% | 11.8% | 12.3% |
| | Main peak: 55.3% | 54.8% | 54.3% | 52.4% |
| | Basic peak: 32.3% | 32.3% | 33.9% | 35.3% |
| NR-CE-SDS | Monomer: 97.1% | 97.1% | 96.6% | 96.5% |
| | 2HC1LC: 2.6% | 1.6% | 1.9% | 2.2% |
| | 1HC1LC: not detected | 0.2% | 0.3% | 0.1% |
| R-CE-SDS | LC+HC: 98.4% | 98.4% | 98.1% | 98.5% |
| | NGHC: 1.2% | 1.2% | 1.2% | 1.2% |
| DAR distribution | DAR value: 2.0 | 2.0 | 2.0 | 2.0 |
| | DAR2: 76.8% | 75.6% | 74.7% | 76.7% |
| Amount of unconjugated antibody | 0.50% | 0.40% | 0.71% | < 1.2% |
| Content of free LND1002 | < 0.004% | < 0.004% | < 0.004% | < 0.004% |
| Content of free MMAE | 0.000019% | 0.000044% | 0.000055% | 0.000067% |
| Antigen binding activity | 105% | 103% | 102% | 115% |
| Biological activity | 103% | 100% | 100% | 98% |

**Table 25: Stability results of key quality indicators of batch 1 under GMP**

| Investigation item | Batch 1 | | | |
|---|---|---|---|---|
| | 0 month | 5°C ± 3°C | | |
| | | 12 months | 24 months | 30 months |
| SEC-HPLC | HMWS: 0.9% | 0.6% | 0.9% | 0.8% |
| | Monomer: 98.4% | 98.1% | 97.7% | 97.4% |
| | LMWS: 0.7% | 1.3% | 1.4% | 1.7% |
| CEX-HPLC | Acid peak: 12.4% | 12.3% | 12.8% | 13.2% |
| | Main peak: 55.3% | 54.3% | 53.3% | 51.9% |
| | Basic peak: 32.3% | 33.5% | 33.9% | 34.9% |
| NR-CE-SDS | Monomer: 97.1% | 96.1% | 95.5% | 95.5% |
| | 2HC1LC: 2.6% | 2.0% | 2.1% | 2.2% |
| | 1HC1LC: not detected | 0.2% | 0.4% | 0.1% |
| R-CE-SDS | LC+HC: 98.4% | 98.0% | 97.9% | 98.2% |
| | NGHC: 1.2% | 1.3% | 1.3% | 1.3% |
| DAR | DAR value: 2.0 | 2.0 | 2.0 | 2.0 |

| Investigation item distribution | Batch 1 | | | |
|---|---|---|---|---|
| | 0 month | 5°C ± 3°C | | |
| | | 12 months | 24 months | 30 months |
| | DAR2: 76.8% | 75.1% | 74.8% | 76.8% |
| Amount of unconjugated antibody | 0.50% | 0.40% | 0.75% | < 1.2% |
| Content of free LND1002 | < 0.004% | < 0.004% | < 0.004% | < 0.004% |
| Content of free MMAE | 0.000019% | 0.000583% | 0.0013% | 0.0014% |
| Antigen binding activity | 105% | 105% | 86% | 100% |
| Biological activity | 103% | 99% | 98% | 98% |

**Table 26: Stability results of key quality indicators of batch 2 under GMP**

| Investigation item | Batch 2 | | | |
|---|---|---|---|---|
| | 0 month | - 20°C ± 5°C | | |
| | | 12 months | 24 months | 30 months |
| SEC-HPLC | HMWS: 0.6% | 0.7% | 0.7% | 0.8% |
| | Monomer: 97.4% | 97.8% | 97.6% | 98.2% |
| | LMWS: 2.0% | 1.5% | 1.8% | 1.1% |
| CEX-HPLC | Acid peak: 11.7% | 15.6% | 12.8% | 12.4% |
| | Main peak: 59.4% | 57.5% | 57.1% | 55.5% |
| | Basic peak: 28.9% | 26.8% | 30.1% | 32.1% |
| NR-CE-SDS | Monomer: 97.1% | 96.3% | 94.8% | 95.6% |
| | 2HC1LC: 1.1% | 1.5% | 3.6% | 2.4% |
| | 1HC1LC: 0.1% | 0 | 0.11% | 0.13% |
| R-CE-SDS | LC+HC: 98.5% | 98.3% | 98.4% | 98.1% |
| | NGHC: 1.1% | 1.2% | 1.2% | 1.3% |
| DAR distribution | DAR value: 2.0 | 2.0 | 2.0 | 2.0 |
| | DAR2: 77.7% | 76.4% | 76.6% | 77.5% |
| Amount of unconjugated antibody | 0.47% | 0.43% | 0.41% | < 1.2% |
| Content of free LND1002 | < 0.004% | < 0.004% | < 0.004% | < 0.004% |
| Content of free MMAE | 0.000018% | 0.000037% | 0.000026% | 0.000038% |
| Antigen binding activity | 89% | 98% | 90% | 95% |
| Biological activity | 105% | 102% | 104% | 108% |

**Table 27: Stability results of key quality indicators of batch 2 under GMP**

| Investigation item | Batch 2 | | | |
|---|---|---|---|---|
| | 0 month | 5°C ± 3°C | | |
| | | 12 months | 24 months | 30 months |
| SEC-HPLC | HMWS: 0.6% | 0.7% | 0.6% | 0.7% |
| | Monomer: 97.4% | 97.0% | 96.4% | 96.8% |
| | LMWS: 2.0% | 2.3% | 3.0% | 2.5% |
| CEX-HPLC | Acid peak: 11.7% | 15.4% | 13.5% | 12.9% |
| | Main peak: 59.4% | 57.7% | 56.3% | 55.4% |
| | Basic peak: 28.9% | 26.9% | 30.2% | 31.7% |
| NR-CE-SDS | Monomer: 97.1% | 95.1% | 93.4% | 94.2% |
| | 2HC1LC: 1.1% | 2.0% | 3.8% | 2.5% |
| | 1HC1LC: 0.1% | 0.1% | 0.11% | 0.15% |
| R-CE-SDS | LC+HC: 98.5% | 98.1% | 98.2% | 97.7% |
| | NGHC: 1.1% | 1.2% | 1.2% | 1.4% |
| DAR distribution | DAR value: 2.0 | 2.0 | 2.0 | 2.0 |
| | DAR2: 77.7% | 76.3% | 76.6% | 77.6% |
| Amount of unconjugated antibody | 0.47% | 0.47% | 0.53% | < 1.2% |
| Content of free LND1002 | < 0.004% | < 0.004% | < 0.004% | < 0.004% |
| Content of free MMAE | 0.000018% | 0.000470% | 0.00052% | 0.00098% |
| Antigen binding activity | 89% | 94% | 106% | 102% |
| Biological activity | 105% | 97% | 105% | 103% |

**Table 28: Stability results of key quality indicators of batch 3 under GMP**

| Investigation item | Batch 3 | | | |
|---|---|---|---|---|
| | 0 month | - 20°C ± 5°C | | |
| | | 12 months | 24 months | 30 months |
| SEC-HPLC | HMWS: 0.7% | 0.8% | 0.8% | 0.8% |
| | Monomer: 97.6% | 97.7% | 97.6% | 98.1% |
| | LMWS: 1.7% | 1.5% | 1.6% | 1.1% |
| CEX-HPLC | Acid peak: 17.1% | 15.8% | 13.2% | 13.2% |
| | Main peak: 54.9% | 56.7% | 55.5% | 54.8% |
| | Basic peak: 28.0% | 27.5% | 31.3% | 32.0% |
| NR-CE-SDS | Monomer: 96.3% | 96.3% | 94.7% | 95.6% |
| | 2HC1LC: 1.8% | 1.5% | 3.8% | 2.4% |
| | 1HC1LC: 0.1% | 0 | 0.09% | 0.16% |
| R-CE-SDS | LC+HC: 98.2% | 98.2% | 98.1% | 98.0% |
| | NGHC: 1.3% | 1.2% | 1.2% | 1.3% |
| DAR distribution | DAR value: 2.0 | 2.0 | 2.0 | 2.0 |
| | DAR2: 78.0% | 76.1% | 76.2% | 77.1% |
| Amount of unconjugated antibody | 0.47% | 0.51% | 0.50% | < 1.2% |
| Content of free LND1002 | < 0.004% | < 0.004% | < 0.004% | < 0.004% |
| Content of free MMAE | 0.000023% | 0.000041% | 0.000022% | 0.00004% |
| Antigen binding activity | 108% | 109% | 98% | 96% |
| Biological activity | 107% | 98% | 105% | 103% |

**Table 29: Stability results of key quality indicators of batch 3 under GMP**

| Investigation item | Batch 3 | | | |
|---|---|---|---|---|
| | 0 month | 5°C ± 3°C | | |
| | | 12 months | 24 months | 30 months |
| SEC-HPLC | HMWS: 0.7% | 0.8% | 0.7% | 0.8% |
| | Monomer: 97.6% | 97.2% | 96.5% | 96.8% |
| | LMWS: 1.7% | 2.1% | 2.8% | 2.4% |
| CEX-HPLC | Acid peak: 17.1% | 15.8% | 13.7% | 13.6% |
| | Main peak: 54.9% | 57.3% | 55.4% | 54.8% |
| | Basic peak: 28.0% | 26.9% | 30.8% | 31.6% |
| NR-CE-SDS | Monomer: 96.3% | 95.9% | 93.8% | 94.4% |
| | 2HC1LC: 1.8% | 1.5% | 3.8% | 2.4% |
| | 1HC1LC: 0.1% | 0 | 0.10% | 0.11% |
| R-CE-SDS | LC+HC: 98.2% | 98.1% | 98.0% | 97.6% |
| | NGHC: 1.3% | 1.2% | 1.2% | 1.4% |
| DAR distribution | DAR value: 2.0 | 2.0 | 2.0 | 2.0 |
| | DAR2: 78.0% | 76.0% | 76.3% | 77.2% |
| Amount of unconjugated antibody | 0.47% | 0.57% | 0.59% | < 1.2% |
| Content of free LND1002 | < 0.004% | < 0.004% | < 0.004% | < 0.004% |
| Content of free MMAE | 0.000023% | 0.000511% | 0.00052% | 0.0010% |
| Antigen binding activity | 108% | 95% | 109% | 114% |
| Biological activity | 107% | 108% | 101% | 106% |

The above-mentioned research results show that in the low-temperature freezing state, until 30 months, there is no significant change in the physical and chemical properties of the antibody moiety, which is consistent with the stability trend of the liquid preparation of the antibody, and there is no significant change in both DAR value and DAR2 distribution of the ADC moiety, indicating that the quality of the conjugate is uniform during storage; although the free drug sheds, the shedding amount is very low, wherein the content of free LND1002 is less than 0.004%, and the content of free MMAE is less than 0.0015%; the product can be stored for a long time, and the antigen binding activity and the biological activity do change with the change in storage conditions; and according to the data of different batches, the consistency among batches is good, and the long-term stability is good. In summary, the ideal technical effects are achieved.

**Table 30: Related sequences of antibody involved in the present application**

| **Antibody region/classif ication** | **IMGT CDR definition:** |
|---|---|
| FR-H1 | EVQLSESGGALVQPGESLRLSCAAS (SEQ ID NO: 11) |
| CDR-H1 | GFTFSSYA (SEQ ID NO: 1) |
| FR-H2 | MTWVRQAPGKGLEWVSS (SEQ ID NO: 12) |
| CDR-H2 | LSGSGRST (SEQ ID NO: 2) |
| FR-H3 | YYAASIKGRFTISRDNSKNTLYLQMSSLRAEDTAIYYC (SEQ ID NO: 13) |
| CDR-H3 | AKSLSYYHYYFDY (SEQ ID NO: 3) |
| FR-H4 | WGQGTLVTVSS (SEQ ID NO: 14) |
| FR-L1 | DIQLTQSPSFLSASVGDRVPITCRAS (SEQ ID NO: 15) |
| CDR-L1 | QDISNY (SEQ ID NO: 4) |
| FR-L2 | LAWYQQKPGKAPKLLIY (SEQ ID NO: 16) |
| CDR-L2 | SAS (SEQ ID NO: 5) |
| FR-L3 | TLQSGVPSRFSGSGSGTEFTLTISSLQPEDFASYHC (SEQ ID NO: 17) |
| CDR-L3 | QQVKTYPLT (SEQ ID NO: 6) |
| FR-L4 | FGGGTKVEIK (SEQ ID NO: 18) |
| HV (heavy chain variable region) | EVQLSESGGALVQPGESLRLSCAASGFTFSSYAMTWVRQAPGKGLEWVSSLS GSGRSTYYAASIKGRFTISRDNSKNTLYLQMSSLRAEDTAIYYCAKSLSYYHY YFDYWGQGTLVTVSS (SEQ ID NO: 7) |
| LV (light chain variable region) | DIQLTQSPSFLSASVGDRVPITCRASQDISNYLAWYQQKPGKAPKLLIYSASTL QSGVPSRFSGSGSGTEFTLTISSLQPEDFASYHCQQVKTYPLTFGGGTKVEIK (SEQ ID NO: 8) |
| HC (heavy chain) | EVQLSESGGALVQPGESLRLSCAASGFTFSSYAMTWVRQAPGKGLEWVSSLS GSGRSTYYAASIKGRFTISRDNSKNTLYLQMSSLRAEDTAIYYCAKSLSYYHY YFDYWGQGTLVTVSSASTKGPSVFPLAPSSKSTSGGTAALGCLVKDYFPEPVT VSWNSGALTSGVHTFPAVLQSSGLYSLSSVVTVPSSSLGTQTYICNVNHKPSNT KVDKKVEPKSCDKTHTCPPCPAPELLGGPSVFLFPPKPKDTLMISRTPEVTCVV VDVSHEDPEVKFNWYVDGVEVHNAKTKPREEQYNSTYRVVSVLTVLHQDWL NGKEYKCKVSNKALPAPIEKTISKAKGQPREPQVYTLPPSRDELTKNQVSLTCL VKGFYPSDIAVEWESNGQPENNYKTTPPVLDSDGSFFLYSKLTVDKSRWQQG NVFSCSVMHEALHNHYTQKSLSLSPGK (SEQ ID NO: 9) |
| LC (light chain) | DIQLTQSPSFLSASVGDRVPITCRASQDISNYLAWYQQKPGKAPKLLIYSASTL QSGVPSRFSGSGSGTEFTLTISSLQPEDFASYHCQQVKTYPLTFGGGTKVEIKRT VAAPSVFIFPPSDEQLKSGTASVVCLLNNFYPREAKVQWKVDNALQSGNSQES VTEQDSKDSTYSLSSTLTLSKADYEKHKVYACEVTHQGLSSPVTKSFNRGEC (SEQ ID NO: 10) |
| Amino acid sequence of transglutamin ase | DSDERVTPPAEPLDRMPDPYRPSYGRAETIVNNYIRKWQQVYSHRDGRKQQM TEEQREWLSYGCVGVTWVNSGQYPTNRLAFAFFDEDKYKNELKNGRPRSGET RAEFEGRVAKDSFDEAKGFQRARDVASVMNKALENAHDEGAYLDNLKKELA NGNDALRNEDARSPFYSALRNTPSFKDRNGGNHDPSKMKAVIYSKHFWSGQD RSGSSDKRKYGDPEAFRPDRGTGLVDMSRDRNIPRSPTSPGESFVNFDYGWFG AQTEADADKTVWTHGNHYHAPNGSLGAMHVYESKFRNWSDGYSDFDRGAY VVTFVPKSWNTAPDKVTQGWP (SEQ ID NO: 19) |

## Claims

**1.** A pharmaceutical composition, comprising an anti-CLDN18.2 antibody-drug conjugate, a buffer, a stabilizer, and a surfactant, wherein the antibody comprises a heavy chain comprising HCDR1 to HCDR3 having amino acid sequences of SEQ ID NOs: 1, 2, and 3, respectively, and/or a light chain comprising LCDR1 to LCDR3 having amino acid sequences of SEQ ID NOs: 4, 5, and 6, respectively, and wherein the structure of the drug-linker moiety (L&D) of the antibody-drug conjugate is:

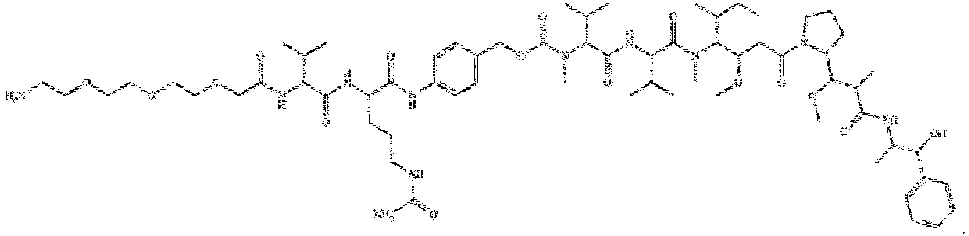

**2.** The pharmaceutical composition according to claim 1, wherein the pharmaceutical composition has one or more of the following characteristics:
(a) the surfactant is polysorbate 20 or polysorbate 80, preferably polysorbate 20;
(b) the stabilizer is sucrose or trehalose, preferably sucrose;
(c) the buffer is histidine-histidine hydrochloride, succinic acid-sodium succinate, or citric acid-sodium citrate, preferably histidine-histidine hydrochloride.

**3.** The pharmaceutical composition according to claim 1 or 2, wherein the pharmaceutical composition comprises:
5-30 mM histidine-histidine hydrochloride,
0.02 wt% to 0.08 wt% of polysorbate 20 or polysorbate 80,
6 wt% to 8 wt% of sucrose, and
5-50 mg/ml of the antibody-drug conjugate,
and has a pH of 5.0-6.5.

**4.** The pharmaceutical composition according to claim 1 or 2, wherein the pharmaceutical composition comprises:
10-30 mM histidine-histidine hydrochloride,
0.02 wt% to 0.04 wt% of polysorbate 20 or polysorbate 80,
6 wt% to 7 wt% of sucrose, and
10-20 mg/ml of the antibody-drug conjugate,
and has a pH of 5.0-6.0.

**5.** The pharmaceutical composition according to claim 1 or 2, wherein the pharmaceutical composition comprises:
20 mM histidine-histidine hydrochloride,
0.02 wt% of polysorbate 20,
6 wt% of sucrose, and
10 mg/ml of the antibody-drug conjugate,
and has a pH of 5.5.

**6.** The pharmaceutical composition according to any one of claims 1-5, wherein the pharmaceutical composition further comprises water.

**7.** The pharmaceutical composition according to any one of claims 1-6, wherein the pharmaceutical composition is a liquid preparation, a freeze-dried preparation, or a powder injection preparation, preferably a liquid preparation.

**8.** The pharmaceutical composition according to any one of claims 1-7, wherein the heavy chain of the antibody comprises a heavy chain variable region having an amino acid sequence of SEQ ID NO: 7, and/or the light chain of the antibody comprises a light chain variable region having an amino acid sequence of SEQ ID NO: 8.

**9.** The pharmaceutical composition according to any one of claims 1-8, for use in the treatment of a cancer, preferably a CLDN18.2-positive cancer.

**10.** The pharmaceutical composition according to claim 9, wherein the cancer is selected from a group consisting of: breast cancer, gastric cancer, colorectal cancer (including colon cancer and rectal cancer), lung cancer (including small cell lung cancer and non-small cell lung cancer), esophageal cancer, salivary gland cancer, esophagogastric junction adenocarcinoma, cholangiocarcinoma, Paget's disease, pancreatic cancer, ovarian cancer, uterine carcinosarcoma, urothelial carcinoma, prostate cancer, bladder cancer, gastrointestinal stromal tumor, cervical cancer, squamous cell carcinoma, peritoneal cancer, liver cancer, hepatocellular carcinoma, colon cancer, rectal cancer, endometrial cancer, uterine cancer, kidney cancer, vulvar cancer, thyroid cancer, penile cancer, leukemia, malignant lymphoma, plasmacytoma, myeloma, neuroepithelial tissue tumor, schwannoma, head and neck cancer, skin cancer, laryngeal cancer, gallbladder cancer, cholangiocarcinoma, mesothelioma, and sarcoma.

**11.** The pharmaceutical composition according to claim 9, wherein the cancer is selected from a group consisting of: breast cancer, gastric cancer, colorectal cancer, non-small cell lung cancer, esophageal cancer, salivary gland cancer, esophagogastric junction adenocarcinoma, cholangiocarcinoma, Paget's disease, pancreatic cancer, ovarian cancer, and uterine carcinosarcoma.

**12.** The pharmaceutical composition according to claim 9, wherein the cancer is selected from a group consisting of: breast cancer, gastric cancer, colorectal cancer, non-small cell lung cancer, esophageal cancer, salivary gland cancer, esophagogastric junction adenocarcinoma, cholangiocarcinoma, and Paget's disease.

**13.** The pharmaceutical composition according to claim 9, wherein the cancer is selected from a group consisting of: breast cancer, gastric cancer, colorectal cancer, and non-small cell lung cancer.

**14.** The pharmaceutical composition according to any one of claims 9-13, wherein the pharmaceutical composition is for administration in combination with other therapies, such as a surgery, a radiation therapy, or a hormonal therapy.

**15.** The pharmaceutical composition according to any one of claims 9-13, wherein the pharmaceutical composition is for administration in combination with other anti-cancer agents.

**16.** The pharmaceutical composition according to claim 15, wherein the other anti-cancer agents are selected from a group consisting of: 5-fluorouracil (5-FU), pertuzumab, trastuzumab, paclitaxel, carboplatin, cisplatin, gemcitabine, capecitabine, irinotecan (CPT-11), docetaxel, pemetrexed, sorafenib, vinblastine, vinorelbine, everolimus, tanespimycin, bevacizumab, oxaliplatin, and lapatinib.

**17.** Use of the pharmaceutical composition according to any one of claims 1-8 in the manufacture of a medicament for the treatment of a cancer, wherein the cancer is preferably a CLDN18.2-positive cancer.

**18.** The use according to claim 17, wherein the cancer is selected from a group consisting of: breast cancer, gastric cancer, colorectal cancer (including colon cancer and rectal cancer), lung cancer (including small cell lung cancer and non-small cell lung cancer), esophageal cancer, salivary gland cancer, esophagogastric junction adenocarcinoma, cholangiocarcinoma, Paget's disease, pancreatic cancer, ovarian cancer, uterine carcinosarcoma, urothelial carcinoma, prostate cancer, bladder cancer, gastrointestinal stromal tumor, cervical cancer, squamous cell carcinoma, peritoneal cancer, liver cancer, hepatocellular carcinoma, colon cancer, rectal cancer, endometrial cancer, uterine cancer, kidney cancer, vulvar cancer, thyroid cancer, penile cancer, leukemia, malignant lymphoma, plasmacytoma, myeloma, neuroepithelial tissue tumor, schwannoma, head and neck cancer, skin cancer, laryngeal cancer, gallbladder cancer, cholangiocarcinoma, mesothelioma, and sarcoma.

**19.** The use according to claim 17, wherein the cancer is selected from a group consisting of: breast cancer, gastric cancer, colorectal cancer, non-small cell lung cancer, esophageal cancer, salivary gland cancer, esophagogastric junction adenocarcinoma, cholangiocarcinoma, Paget's disease, pancreatic cancer, ovarian cancer, and uterine carcinosarcoma.

**20.** The use according to claim 17, wherein the cancer is selected from a group consisting of: breast cancer, gastric cancer, colorectal cancer, non-small cell lung cancer, esophageal cancer, salivary gland cancer, esophagogastric junction adenocarcinoma, cholangiocarcinoma, and Paget's disease.

**21.** The use according to claim 17, wherein the cancer is selected from a group consisting of: breast cancer, gastric cancer, colorectal cancer, and non-small cell lung cancer.

**22.** The use according to any one of claims 17-21, wherein the pharmaceutical composition is for administration in combination with other therapies, such as a surgery, a radiation therapy, or a hormonal therapy.

**23.** The use according to any one of claims 17-21, wherein the pharmaceutical composition is for administration in combination with other anti-cancer agents.

**24.** The use according to claim 23, wherein the other anti-cancer agents are selected from a group consisting of: 5-fluorouracil (5-FU), pertuzumab, trastuzumab, paclitaxel, carboplatin, cisplatin, gemcitabine, capecitabine, irinotecan (CPT-11), docetaxel, pemetrexed, sorafenib, vinblastine, vinorelbine, everolimus, tanespimycin, bevacizumab, oxaliplatin, and lapatinib.

**24.** A method for treating a cancer, comprising administering to an individual having a cancer in need of treatment an effective amount of the pharmaceutical composition according to any one of claims 1-8, wherein the cancer is preferably a CLDN18.2-positive cancer.

**25.** The method according to claim 24, wherein the cancer is selected from a group consisting of: breast cancer, gastric cancer, colorectal cancer (including colon cancer and rectal cancer), lung cancer (including small cell lung cancer and non-small cell lung cancer), esophageal cancer, salivary gland cancer, esophagogastric junction adenocarcinoma, cholangiocarcinoma, Paget's disease, pancreatic cancer, ovarian cancer, uterine carcinosarcoma, urothelial carcinoma, prostate cancer, bladder cancer, gastrointestinal stromal tumor, cervical cancer, squamous cell carcinoma, peritoneal cancer, liver cancer, hepatocellular carcinoma, colon cancer, rectal cancer, endometrial cancer, uterine cancer, kidney cancer, vulvar cancer, thyroid cancer, penile cancer, leukemia, malignant lymphoma, plasmacytoma, myeloma, neuroepithelial tissue tumor, schwannoma, head and neck cancer, skin cancer, laryngeal cancer, gallbladder cancer, cholangiocarcinoma, mesothelioma, and sarcoma.

**26.** The method according to claim 24, wherein the cancer is selected from a group consisting of: breast cancer, gastric cancer, colorectal cancer, non-small cell lung cancer, esophageal cancer, salivary gland cancer, esophagogastric junction adenocarcinoma, cholangiocarcinoma, Paget's disease, pancreatic cancer, ovarian cancer, and uterine carcinosarcoma.

**27.** The method according to claim 24, wherein the cancer is selected from a group consisting of: breast cancer, gastric cancer, colorectal cancer, non-small cell lung cancer, esophageal cancer, salivary gland cancer, esophagogastric junction adenocarcinoma, cholangiocarcinoma, and Paget's disease.

**28.** The method according to claim 24, wherein the cancer is selected from a group consisting of: breast cancer, gastric cancer, colorectal cancer, and non-small cell lung cancer.

**29.** The method according to any one of claims 24-28, further comprising administering to the individual other therapies, such as a surgery, a radiation therapy, or a hormonal therapy.

**30.** The method according to any one of claims 24-28, further comprising administering to the individual other anti-cancer agents.

**31.** The method according to claim 30, wherein the other anti-cancer agents are selected from a group consisting of: 5-fluorouracil (5-FU), pertuzumab, trastuzumab, paclitaxel, carboplatin, cisplatin, gemcitabine, capecitabine, irinotecan (CPT-11), docetaxel, pemetrexed, sorafenib, vinblastine, vinorelbine, everolimus, tanespimycin, bevacizumab, oxaliplatin, and lapatinib.

**32.** A method for preparing the pharmaceutical composition according to any one of claims 1 to 8, comprising the following steps:
preparing the anti-CLDN18.2 antibody-drug conjugate,
transferring the anti-CLDN18.2 antibody-drug conjugate to a liquid system containing the other components of the pharmaceutical composition by liquid replacement, and
performing sterile filtration and sterile filling.
